# EUROPEAN PATENT SPECIFICATION

(11) **EP 0 213 272 B1**
(45) Date of publication and mention of the grant of the patent: **09.09.1992**
(21) Application number: 86105955.8
(22) Date of filing: 30.04.1986
(51) Int. Cl.: A61K 31/40, A61K 37/02, A61K 41/00, C07D 487/22

(54) **New tetrapyrrole polyaminomonocarboxylic acid therapeutic agents**
Tetrapyrrolpolyaminomonocarbonsäure-Therapiemittel
Agents thérapeutiques à base d'acides tétrapyrrole polyaminomonocarboxyliques

(30) Priority: 30.04.1985 US 728752
(43) Date of publication of application: 11.03.1987
(73) Proprietor: Nippon Petrochemicals Co., Ltd., Tokyo (JP)
(72) Inventor: Bommer, Jerry C., Ogden Utah (US); Burnham, Bruce F., Logan Utah (US)
(74) Representative: Lehn, Werner, Dipl.-Ing.

(56) References cited:
- EP-A- 0 142 732
- EP-A- 0 168 831
- EP-A- 0 168 832
- WO-A-84/01382
- DE-A- 2 809 093
- US-A- 4 393 071

## Description

This invention relates to new therapeutic compositions which are useful in photodiagnosis and phototherapy, especially in the detection and treatment of tumors and cancerous tissues in the human or animal body.

It is known to irradiate tumors and cancerous tissues in the human body with intensive light following administration of a hematoporphyrin derivative in the wavelength range of 626 to 636 namometers to reduce and, at times, destroy the cancerous cells (see PCT published specification WO 83/00811). It is also known that porphyrins, especially the sodium salt of protoporphyrins, can maintain or promote the normal functions of cells and are useful for preventing the genesis, growth, metastasis, and relapse of malignant tumors. Japanese Published Patent Application No. 125737/76 describes the use of porphyrins as tumor inhibiting agents, exemplifying etioporphyrin, mesoporphyrin, protoporphyrin, deuteroporphyrin, hematoporphyrin, coproporphyrin, and uroporphyrin.

In Tetrahedron Letters No. 23, pp. 2017 - 2020 (1978) describes an amino monocarboxylic acid adduct of the pigment bonellin obtained by extraction of principally the body wall of the marine echuroid B. viridis. The structure of these adducts is presumed to be an amide formed through either of the free carboxy groups of bonellin and the amino mono-carboxylic acid. Hydrolysis of the adduct yielded a mixture of valine, isoleucine, leucine and alloisoleucine. No use for these amino acid adducts is described in this reference.

That the tetrapyrroles cause intense photosensitivity in animals in well known and has been documented in numerous articles in literature, e.g., J. lntr. Sci. Vitaminol, 27, 521-527 (1981); Agric. Biol. Chem., 46(9), 2183-2193 (1982); Chem. Abst. 98, 276 (1983) and 88 6976m (1928).

EP-A-0142732 describes pheophorbide derivatives and alkali metal salts thereof as useful compounds in cancer treatment.

The therapeutic agents contemplated by this invention are cyclic tetrapyrroles derived by various procedures from naturally-occurring tetrapyrroles. The cyclic tetrapyrroles have as their common parent tetrapyrrole, uroporphyrinogen, and possess the following ring structure: 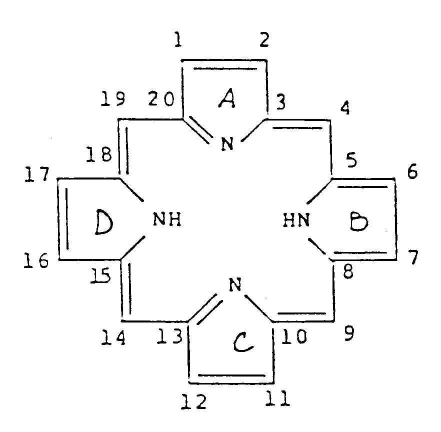 in which the positions in the molecule are numbered 1-20, and the rings identified by letters A, B, C and D, and also include perhydro-, e.g., dihydro- and tetrahydro-, derivatives of the said ring structure, e.g., compounds in which one or more double bonds are absent. There are present in the ring system four pyrrole rings joined through the alpha positions of the respective pyrrole rings by a methine group, i.e., -CH =. The compounds of the present invention are designated as derivatives of the tetrapyrroles for convenience in the disclosure and the appended claims and it will be understood that the term "tetrapyrrole" will designated compounds of the characteristic ring structure designated hereinbefore as well as the corresponding perhydro derivatives.

The tetrapyrroles employed in the present invention are all derived by various means and various alteration procedures from natural tetrapyroles. The naturally occurring tetrapyrroles have as their common ancestor uroporphyrinogen III, a hexahydroporphyrin reduced at the bridge positions. For example, synthetic or biosynthetic derivatives or products of protoporphyrins IX or protoporphyrinogen IX are well-known in the art (see, for example, Porphyrins and Metalloporphyrins, K. Smith Elsivier; The Porphyrins (Vols. 1-7) D. Dolphin, Academic Press; and Biosynthetic Pathways, Vol. III, Chapter by B. Burnham, editor D.M. Greenberg, Academic Press).

The non-cyclic tetrapyrroles are commonly known as bile pigments and include, for example, bilirubin and biliverdin. These tetrapyrroles are also derived from protoporphyrin, e.g., as metabolic products in animals.

A further characteristic of the present new therapeutic composition is the presence of at least one amide linkage in a substituent at any of the numbered positions of the ring structure. These are present in the instant new compounds together with other substituents as defined hereinafter.

Subject matter of the present invention is therefore a therapeutic composition according to claim 1.

Preferred embodiments of this composition are subject matter of claims 2 to 5.

Further subject matter is the use according to claim 6.

Thus, the present invention contemplates the therapeutic compositions containing amino acid or peptide derivatives of compounds which contain the chromophore of porphyrins, chlorins or bacteriochlorins, as well as related porphyrin compounds. The peptide linkage involves a carboxy group of the chromophore-bearing compound and the amino group of the specified amino acid. The present new therapeutic compositions embrace, inter alia, derivatives of the tetrapyrroles which contain a free carboxy group. These derivatives include the major classes of tetrapyrroles: carboxy-containing porphyrins, chlorins, and bacteriochlorins, which are well-known to those skilled in this art.

The amino acid employed in the present invention to form the aforesaid peptide linkage are aminomonocarboxylic acids in which the amino group, of course, is located on a carbon atom of the monocarboxylic acid. The specific position of the amino group in the carbon atom chain is not critical, the only requirement that the amino group be available to form the requisite peptide linkage with the carboxyl group of the selected porphyrin. Thus, a variety of amino monocarboxylic acids are useful in the present invention, including serine, glycine, a-aminoalanine, ,8-aminoalanine, e-amino-n-caproic acid, piperidine-2-carboxylic acid, piperidine-6-carboxylic acid, pyrrole-2-carboxylic acid, pyrrole-5-carboxylic acid, piperidine-6-propionic acid or pyrrole-5-acetic acid. These amino acids may be substituted with angular alkyl groups, such as methyl and ethyl groups, as well as other groups which do not adversely affect the capability of the amino group to form the peptide linkage, e.g., alkoxy groups, or acyloxy groups, and may also include additional amino groups. The preferred amino acids are the naturally occurring a-amino acids, serine, alanine, and glycine, which are readily available and up to the present, have provided the best results.

Exemplary compounds of the tetrapyrrole classes are illustrated in Table I in which the numbered positions of the tetrapyrrole ring structure are used to designate the position of the indicated substituent. The absence of double bonds in the ring system is designated under "dihydro" with each set of numbers (ring position) indicating the absence of a double bond between the designated positions.

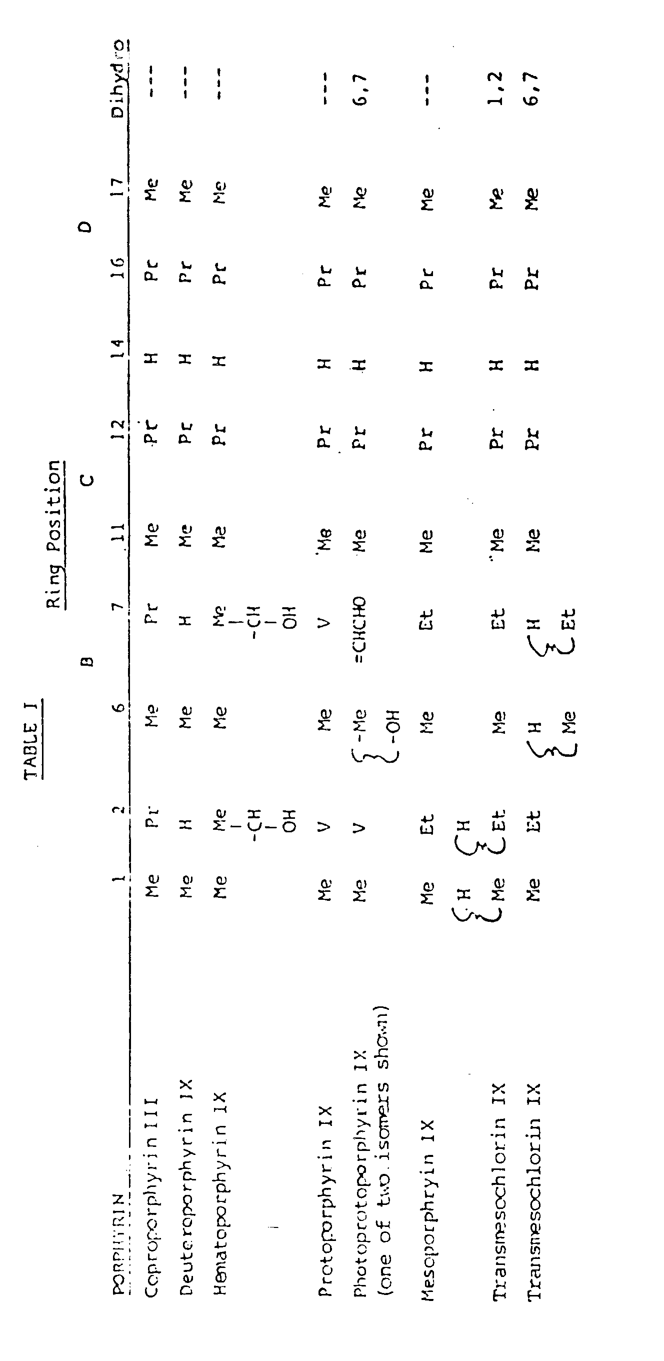

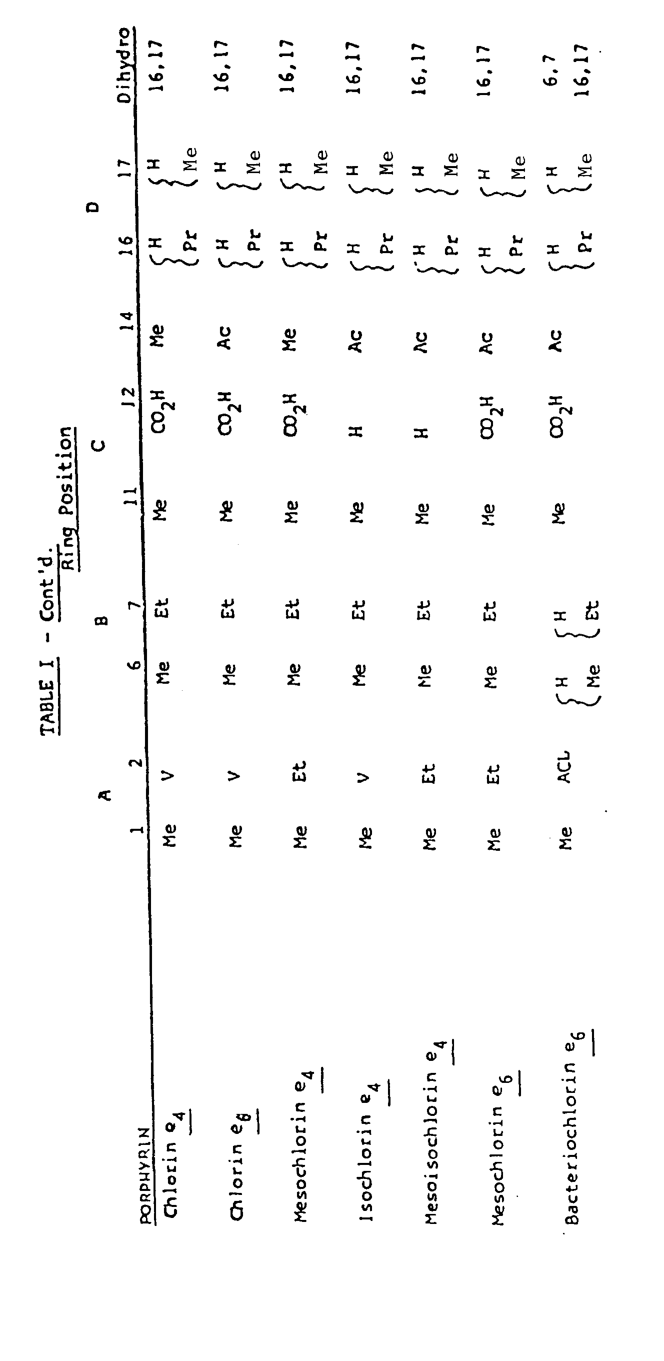

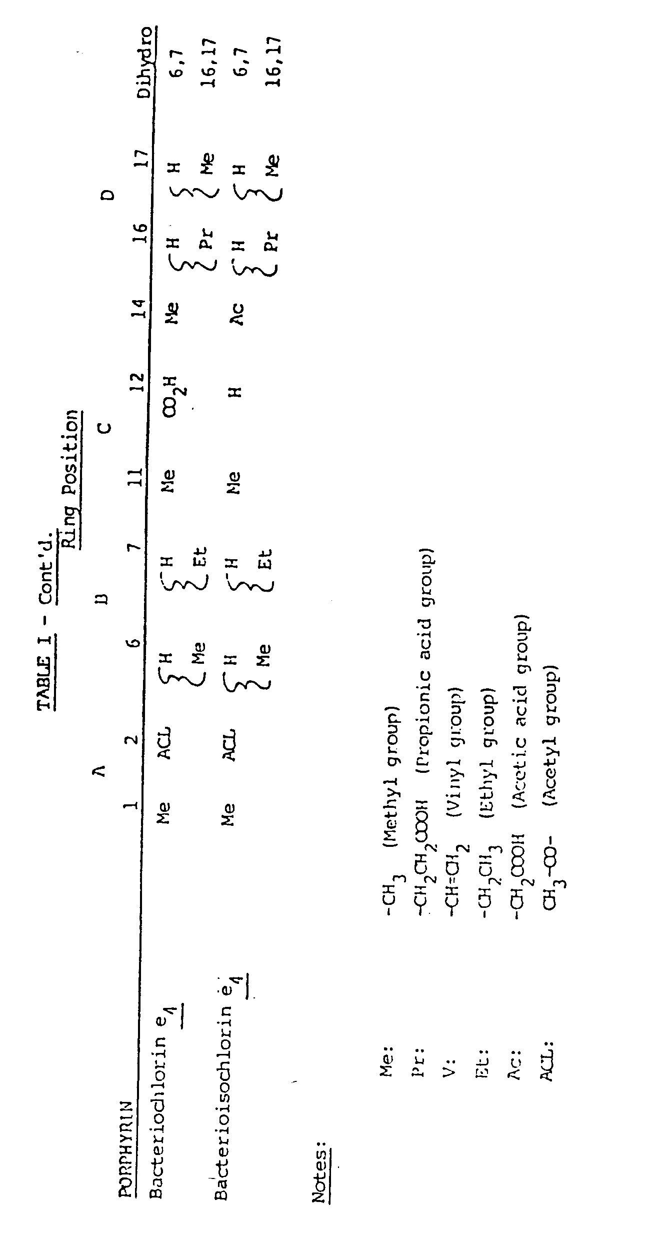

The preferred tetrapyrrole carboxylic acids are those wherein at least three carboxylic acid groups are present in the tetrapyrrole, preferably asymmetrically attached to the porphyrin ring system, e.g., the carboxylic acid groups are present on the rings A and B side of the molecule or on the rings D and C side of the molecule.

The particularly preferred therapeutic compositions comprise a fluorescent mono, di, or polyamide of an aminomonocarboxylic acid and a tetrapyrrole of the formula: 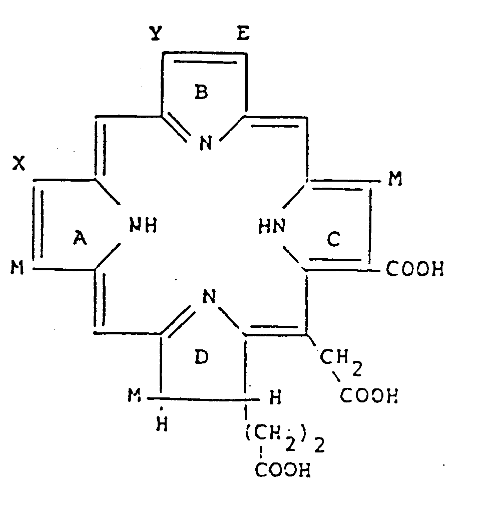 wherein;
X = H, vinyl, ethyl, acetyl or formyl;
Y = methyl or formyl;
M = methyl; and
E = ethyl

and pharmaceutically-acceptable salts thereof.

The compounds of the therapeutic composition form salts with either acids or bases. The acid salts are particularly useful for purification and/or separation of the final amide products as are the salts formed with bases. The base salts, however, are particularly preferred for diagnostic and therapeutic use as herein- described.

The acid salts are formed with a variety of acids such as the mineral acids, hydrochloric, hydrobromic, nitric and sulfuric acids, organic acids such as toluenesulfonic and benzenesulfonic acids.

The base salts include, for example, sodium, potassium, calcium, magnesium, ammonium, triethylammonium, trimethylammonium, morpholine or piperidine salts.

The acid and base salts are formed by the simple expediency of dissolving the selected amino acid tetrapyrrole amide in an aqueous solution of the acid or base and evaporation of the solution to dryness. The use of a water-miscible solvent for the amide can assist in dissolving the amide.

The final amide products can also be converted to metal complexes for example by reaction with metal salts. The magnesium complexes may be useful for the same purpose as the adduct product. Other metal complexes, as well as the magnesium complex, including, for example, iron and zinc, are useful to preclude contamination during processing of the adduct product by metals such as nickel, cobalt and copper, which are difficult to remove. Zinc and magnesium are readily removed from the final adduct product after processing is completed.

Since many of the aminomonocarboxylic acids exist in both the D- and L-forms, and also are employed in mixtures of these forms as well as the D,L-form, the selection of the starting amino acid will, of course, result in products in which the respective isomer or mixture of isomers exist. The present invention contemplates the use of all such isomers, but the L-form is particularly preferred.

The present new compounds are prepared by the usual peptide synthetic routes which generally include any amide-forming reaction between the selected amino acid and the specific tetrapyrrole. Thus, any amide-forming derivative of the tetrapyrrole carboxylic acid can be employed in producing the present new peptides, e.g., lower alkyl esters, anhydrides and mixed anhydrides.

The preferred preparative methods use mixed anhydrides of the carboxylic acid or carbodiimides. The reactants are merely contacted in a suitable solvent therefor and allowed to react. Temperatures up to the reflux temperature can be used, with the higher temperatures merely reducing the reaction time. However, excessively high temperatures are usually not preferred so as to avoid unwanted secondary reactions.

The procedures for forming the instant peptides are well known in this art and are provided in detail in the accompanying examples.

Since the selected tetrapyrrole contains more than one carboxyl group, mixtures of products can be formed in-including isomeric di- and even tri- or higher peptide products, depending on the number of carboxyl groups and depending on the selected stoichiometry. Thus, when equivalent mixtures of amino acid and tetrapyrrole are reacted, the product may contain some monopeptides, but also present will be di-or polypeptides. It is generally possible to separate the monopeptides and higher peptides using known chromatographic techniques. However, such separations are not necessary since the mixed peptides are usually comparable to the separated products in their ultimate use. Thus, mixtures of mono, di, and tripeptides of the same tetrapyrrole can be used.

Usually, unreacted tetrapyrrole is separated from the peptide products of the invention during purification as, for example, by chromatographic techniques.

### Photodiagnosis and Phototherapy

The compositions of the present invention are useful for the photodiagnosis and phototherapy of tumor, cancer and malignant tissue (hereinafter referred to as "tumor").

When a man or animal having tumor is treated with doses of a compound of the present invention and when appropriate light rays or electromagnetic waves are applied, the compound emits light, i.e., fluorescence. Thereby the existence, position and size of tumor can be detected, i.e., photo-diagnosis.

When the tumor is irradiated with light of proper wavelength and intensity, the compound is activated to exert a cell killing effect against the tumor. This is called "phototherapy".

Compounds intended for photodiagnosis and phototherapy ideally should have the following properties:
(a) non-toxic at normal therapeutic dosage unless and until activated by light;
(b) should be selectively photoactive;
(c) when light rays or electromagnetic waves are applied, they should emit characteristic and detectable fluorescence;
(d) when irradiated with light rays or electromagnetic waves are applied, they are activated to an extent to exert a cell killing effect against tumor; and
(e) easily metabolized or excreted after treatment.

In accordance with testing up to the present, the present new compounds have the foregoing properties and are also characterized by reasonable solubility in saline at physiological pH.

The compounds in the present composition possess greater fluorescence in tumors than do the corresponding basic tetrapyrroles. Their use provides the best contrast in tumors compared to normal tissue around the tumor. The instant compounds absorb activating energy for phototherapy in the convenient range of 600 to 800 nanometers, with the preferred compounds absorbing in the 620-760 nanometer range, i.e., light of longer wavelengths which more readily permits penetration of energy into the tumor for phototherapeutic purpose.

In present experience, the present compounds more uniformly distribute throughout the tumor than the basic tetrapyrrole permitting the use of considerably lower dosage (to about 1/1 Oth of the required normal dose of the basic tetrapyrrole) which lessens, if not eliminates, photosensitization in the host. They also possess a more consistent fluorescence whereas some of the corresponding tetrapyrroles show inconsistent fluorescence or the fluorescence varies from day to day in the host.

A particularly advantageous property of the present compounds resides in the ease with which they are excreted by the host. Generally, within 48 to 72 hours of intravenous or intraperitoneal administration, there are little or no detectable amounts in normal muscle tissue. The present compounds which are excreted with their chromophore intact are recovered from the feces of the host within 48-72 hours of injection. Under equivalent circumstances, substantial amounts of the corresponding tetrapyrroles remain, as compared with only minor amounts of peptides formed with the aminocarboxylic acids remain in the host, e.g., up to about 20%. This property is extremely important in that it contributes to minimization of photosensitization of the host.

The instant compositions can be used for diagnosis and therapeutic treatment of a broad range of tumors. Examples of tumors are gastric cancer, enteric cancer, lung cancer, breast cancer, uterine cancer, esophageal cancer, ovarian cancer, pancreatic cancer, pharyngeal cancer, sarcomas, hepatic cancer, cancer of the urinary bladder, cancer of the upper jaw, cancer of the bile duct, cancer of the tongue, cerebral tumor, skin cancer, malignant goiter, prostatic cancer, cancer of the parotid gland, Hodgkins's disease, multiple myeloma, renal cancer, leukemia; and malignant lymphocytoma. For diagnosis, the sole requirement is that the tumor be capable of selectivity fluorescing when exposed to proper light. For treatment, the tumor must be penetrable by the activation energy. For diagnosis, light of shorter wavelength is used whereas for therapeutic purposes light of longer wavelength is used to permit ready penetration of the tumor tissue. I hus, tor diagnosis, light ot trom 36U - 76U nanometers can be used, and tor treatment, trom 620 - 760, depending on the individual characteristics of the tetrapyrrole. The absorption characteristics of the present new compounds are substantially the same as the tetrapyrrole from which derived.

It is necessary that the light rays be so intense as to cause the compounds to emit fluorescence for diagnosis and to exert a cell killing effect for therapy.

The source of irradiation for photodiagnosis and phototherapy is not restricted, however, but the laser beam is preferable because intensive light rays in a desired wavelength range can be selectively applied. For example, in photodiagnosis, the compound of the invention is administered to a human or animal body, and after a certain period of time, light rays are applied to the part to be examined. When an endoscope can be used for the affected part, such as lungs, gullet, stomach, womb, urinary bladder or rectum, it is irradiated using the endoscope, and the tumor portion selectively emits fluorescence. This portion is observed visually, or observed through an adapted fiber scope by eye or on a CRT screen.

In phototherapy, after administration of the dosage, the irradiation is carried out by laser beams from the tip of quartz fibers. Besides the irradiation of the surface of tumor, the internal part of the tumor can be irradiated by inserting the tip of quartz fibers into the tumor. The irradiation can be visually observed or imaged on a CRT screen.

For photodiagnosis, light of wavelengths between 360 and 760 nm. is suitable for activating the present tetrapyrrole compounds. Of course, each compound has a specific optimal wavelength of activation. A long wavelength ultraviolet lamp is particularly suitable for photodiagnosis. Similar methods for viewing of the treated tumor can be used as already described for phototherapy.

The dosages of the present compositions will vary depending on the desired effect, whether for diagnosis or for treatment. For diagnosis, doses of as little as 1 mg /kg will be effective, and up to about 20 mg/kg can be used. For treatment, the dose will usually approximate about 0.5 mg/kg. Of course, the dosage for either diagnosis or treatment can be varied widely in view of aforesaid advantageous properties of the present compounds, e.g., the ease of elimination from the host, for one.

The present compositions are apparently non-toxic at the dosage levels employed for diagnosis or treatment. No mortality of test animals due the present compounds has been noted in studies employing dosage levels up to 20 mg/kg.

For both diagnosis and treatment, the present compositions can be administered by the oral, intravenous, or intramuscular routes. They can be formulated as lyophilized sterile, pyrogen-free compounds, preferably in the form of basic salts, e.g., sodium salt. The preferred dosage forms are provided as injectable solutions (isotonic).

The irradiation source used in treatment of tumors containing compounds of this invention is a filtered, high-intensity, continuous source or pumped dye, or other laser and light delivery system, which is capable of performing within the following limits: power intensity 20-500 mw/cm² at wavelengths between 620 and 700 nm. and a total output of at least 500 mw. or greater. Several currently commercially available lasers meet these criteria.

The tetrapyrroles can be prepared by various synthetic methods which are found in the literature, e.g., Pheophorbides
Willstatter, R., Stoll, A.; Investigations on Chlorophyll, (Transl. Schertz, FM.M., Merz, A.R.) p. 249. Science Printing Press, Lancaster, Pennsylvania, 1928.

Pennington, F.C. Strain, H.H., Svec, W.A., Katz, J.J.; J. Amer. Chem. Soc., 86, 1418 (1964).

### Chlorin e₆

Willstatter, R. Stoll, A.; Investigations on Chlorophyll, (Trans., Schertz, F.M., Merz, A.R.,) p. 176. Science Printing Press, Lancaster, Pennsylvania, 1928.

Willstatter, R., Isler, M.; Ann. Chem., 390, 269 (1912).

Fisher, H., Baumler, P.; Ann Chem., 474, 65 (1929).

Fisher, H., Siebel, H.; Ann. Chem., 499, 84 (1932).

Conant, J.B., Mayer, W.W.; J. Amer. Chem. Soc., 52, 3013 (1930).

### Chlorin e₄

Fisher, H., Heckmaier, J., Plotz, E.; Justus Leibigs Ann. Chem., 500, 215 (1933).

Chlorin e₆, e₄, isochlorin e₄.. mesochlorin e₆, bacteriopheophorbide, bacteriochlorin e₆

Fischer and Orth, "Des Chemie des Pyrrole" Akademische Verlazscesellschaft, Leipzig, 1940, Vol. II, Part 2.

### General Reference for Porphyrins

"Porphyrins and Metalloporphyrins" ed. Kevin M. Smith, Elsevier 1975 N.Y.

The compositions of the present invention can be administered to the host in a variety of forms adapted to the chosen route of administration, i.e., orally, intravenously, intramuscularly or subcutaneous routes.

The compositions may be orally administered, for example, with an inert diluent or with an assimilable edible carrier, or it may be enclosed in hard or soft shall gelatin capsule, or it may be compressed into tablets, or it may be incorporated directly with the food of the diet. For oral therapeutic administration, the compositions may be incorporated with excipients and used in the form of ingestible tablets, buccal tablets, troches, capsules, elexirs, suspensions, syrups or wafers. Such compositions and preparations should contain at least 0.1% of active compound. The percentage of the compositions and preparations may, of course, be varied and may conveniently be between about 2 to about 60% of the weight of the unit. The amount of active compound in such therapeutically useful compositions is such that a suitable dosage will be obtained. Preferred compositions or preparations according to the present invention are prepared so that an oral dosage unit form contains between about 50 and 300 mg of active compound.

The tablets, troches, pills or capsules may also contain the following: A binder such as gum tragacanth, acacia, corn starch or gelatin; excipients such as dicalcium phosphate; a disintegrating agent such as corn starch, potato starch or alginic acid; a lubricant such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the compositions, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

The composition may also be administered parenterally or intraperitoneally. Solutions of the composition as a free base or pharmacologically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol or liquid polyethylene glycol), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid or thimerosal. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the composition in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

The present new compositions may also be applied directly to tumors, whether internal or external, in the host in topical compositions. Exemplary compositions include solutions of the new compounds in solvents, particularly aqueous solvents, most preferably water. Alternatively, for topical application particularly to skin tumors, the present new compounds may be dispersed in the usual cream or salve-formulations commonly used for this purpose or may be provided in the form of spray solutions or suspensions which may include a propellant usually employed in aerosol preparations.

As used herein, "pharmaceutically acceptable carrier" includes any and all solvents, dispersion media, coatings, antibacterial and antifungal agents or isotonic and absorption delaying agents. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of tumors in living subjects.

The following examples further illustrate the invention.

### Mono-, di and Triamides:

### EXAMPLE 1

### Di and mono(DL) serinyl mesoporphyrin IX (mixed anhydrides method)

400 mg (0.0007 moles) of mesoporphyrin IX were suspended in 50 ml of tetrahydrofuran (THF). 360 /1.1 (0.0035 moles) of triethylamine were added with stirring. After 10 minutes, 340 µl (0.0031 moles) ethyl chloroformate were added. After stirring 10 minutes, 10 ml (0.01 moles) of 1 M KOH containing 761 mg (0.0072 moles) of DL serine were added to the THF solution. This mixture was stirred 60 minutes at room temperature.

The organic solvent was flashed off and the reaction mixture was checked by silica TLC for product. Benzene/methanol/88% formic acid (8.5/1.5/0.13) was used to develop the chromatrogram.

After checking for product, the solution was adjusted to pH 7.5-8.0 and placed on a reverse phase (C-18 silica) column 2.5 x 30 cm. The reaction mixture was resolved using a linear gradient of 20-70% methanol in 0.01 M KP0₄ buffer pH 6.85 (1 liter total volume).

The column effluent was collected via fraction collector and the tube contents were pooled according to individual components. The order of elution was di-DL-serinyl mesoporphyrin IX, mono-DL-serinyl mesoporphyrin IX and unsubstituted mesoporphyrin IX.

The methanol was flashed off and the material was precipitated at pH 2.5-3.0. The ppt. was washed 3 times with dilute acetic acid in water. The product was dried under vacuum. The yield of di (DL) serinyl mesoporphyrin IX was 95.6 mg.

### EXAMPLE 2

### Di and mono glycyl mesoporphyrin IX (mixed anhydride method)

100 mg (0.000175 moles) of mesoporphyrin IX were suspended in 100 ml of tetrahydrofuran (THF). 360 µl (0.0035 moles) of triethylamine were added with stirring. After 10 minutes, 340 µl (0.0031 moles) of ethylchloroformate were added. After stirring 10 minutes, 10 ml (0.01 moles) of 1 M KOH containing 500 mg (0.0066 moles) of glycine were added to the THF solution. This mixture was stirred 60 minutes at room temperature.

The organic solvent was flashed off and the reaction mixture was checked by silica TLC for product. Benzene/methanol/88% formic acid (8.5/1.5/0/13) was used to develop the chromatogram.

After checking for product, the solution was adjusted to pH 7.5-8.0 and placed on a reverse phase (C-18 silica) column 2.5 x 30 cm. The reaction mixture was resolved using a linear gradient of zero to 50% methanol in 0.01 M KP0₄ buffer pH 6.85 (1 R total volume).

The column effluent was collected in a fraction collector and the contents were sorted according to individual components. The order of elution was diglycyl mesoporphyrin IX, monoglycyl mesoporphyrin IX and unsubstituted mesoporphyrin IX.

The methanol was flashed off and the material was precipitated at pH 2.5-3.0. The ppt. was washed 3 times with dilute acetic acid in water. The product was dried under vacuum.

### EXAMPLE 3

### Di and Mono a DL) alanyl mesoporphyrin IX (mixed anhydride method)

100 mg (0.000175 moles) of mesoporphyrin IX were suspended in 100 ml of tetrahydrofuran (THF). 210 µl (0.002 moles) of triethylamine were added with stirring. After 10 minutes 195 µl (0.00177 moles) of ethylchloroformate were added. After stirring 10 minutes, 10 ml (0.01 moles) of 1 M KOH containing 500 mg (0.0056 moles) of a (DL) alanine were added to the THF solution. This mixture was stirred 60 minutes at room temperature.

The organic solvent was flashed off and the reaction mixture was checked by silica TLC for product. Benzene/methanol/88% formic acid (8.5/1.5/0.13) was used to develop the chromatogram.

After checking for product, the solution was adjusted to pH 7.5-8.0 and placed on a reverse phase (C-18 silica) column 2.5 x 30 cm. The reaction mixture was resolved using a linear gradient of 20-70% methanol in 0.01 M KP0₄ buffer pH 6.85 (1 R total volume).

The column effluent was collected via fraction collection and the tube contents were sorted according to individual components. The order of elution was di-a(DL) alanyl mesoporphyrin IX, mono- a(DL)-alanyl mesoporphyrin IX and unsubstituted mesoporphyrin IX.

The methanol was flashed off and the material was precipitated at pH 2.5-3.0. The ppt. was washed 3 times with dilute acetic acid in water. The product was dried under vacuum.

### EXAMPLE 4

### Di and mono alanyl mesoporphyrin IX (mixed anhydride method)

400 mg (0.0007 moles) of mesoporphyrin IX were suspended in 100 ml of tetrahydrafuran (THF). 360 µl (0.0035 moles) of triethylamine were added with stirring. After 10 minutes, 340 µl (0.0031 moles) ethyl chloroformate were added. After stirring 10 minutes, 10 ml (0.01 moles) of 1 M KOH containing 400 mg (0.0044 moles) of alanine were added to the THF solution. This mixture was stirred 60 minutes at room temperature.

The organic solvent was flashed off and the reaction mixture was checked by silica TLC for product. Benzene/methanol/88% formic acid (8.5/1,5/0.13) was used to develop the chromatogram.

After checking for product, the solution was adjusted to pH 7.5-8.0 and placed on a reverse phase (C-18 silica) column 2.5 x 30 cm. The reaction mixture was resolved using a linear gradient of 40-50% methanol in 0.01 M KP0₄ buffer pH 6.85 (1 R total volume).

The column effluent was collected via fraction collector and the tube contents were pooled according to individual components. The order of elution was di-β-alanyl mesoporphyrin IX, mono-β-alanyl mesoporphyrin IX, and unsubstituted mesoporphyrin IX.

The methanol was flashed off and the material was precipitated at pH 2.5-3.0. The ppt. was washed 3 times with dilute acetic acid in water. The product was dried under vacuum. The yield for di-β-alanyl mesoporphyrin IX was 40 mg: the yield for mono-β-alanyl mesoporphyrin IX was 23 mg.

### EXAMPLE 5

### Di and mono _{E} amino-n-caproyl mesoporphyrin IX (mixed anhydride method)

400mg (0.0007 moles) of mesoporphyrin IX were suspended in 50 ml of tetrahydrofuran (THF). 360 µl (0.0035 moles) of triethylamine were added with stirring. After 10 minutes, 340 µl (0.0031 moles) of ethylchloroformate were added. After stirring 10 minutes, 10 ml (0.01 moles) of 1 M KOH containing 543 mg (0.00414 moles) of e-amino-n-caproic acid were added to the THF solution. This mixture was stirred 60 minutes at room temperature.

The organic solvent was flashed off and the reaction mixture was checked by silica TLC for product. Benzene/methanol/88% formic acid (8.5/1.5/0.13) was used to develop the chromatogram.

After checking for product, the solution was adjusted to pH 7.5-8.0 and placed on a reverse phase (C-18 silica) column 2.5x30 cm. The reaction mixture was resolved using a linear gradient of 20-70% methanol in 0.01 M KP0₄ buffer pH 6.85 (1 R total volume).

The column effluent was collected via fraction collector and the tube contents were pooled according to individual components. I he order ot elution was di-_{E}-amino-n-caproyl mesoporphyrin IX, mono-E-amino-n-caproyl mesoporphyrin IX, and unsubstituted mesoporphyrin IX.

The methanol was flashed off and the material was precipitated at pH 2.5-3.0. The ppt. was washed three times with dilute acetic acid in water. The product was dried under vacuum. The yield of di-e-amino-n-caproyl mesoporphyrin IX was 237 mg.

### EXAMPLE 6

### Di and mono-β-alanyl hematoporphyrin IX (mixed anhydride method)

400 mg (0.00059 moles of hematoporphyrin IX dihydrochloride were suspended in 50 ml of tetrahydrofuran (THF).

360µl (0.0035 moles) of triethylamine were added with stirring. After 10 minutes, 340µl (0.0031 moles) of ethyl chloroformate were added. After stirring 10 minutes, 10 ml (0.01 moles) of 1 M KOH containing 400 mg (0.0044 moles) of alanine were added to the THF solution. This mixture was stirred 60 minutes at room temperature.

The organic solvent was removed by flash evaportaion, keeping the temperature below 50 ° C. The reaction mixture was checked for product by silica TLC using Benzene/methanol/88% formic acid (8.5/1.5/0.13) as solvent to develop the chromatogram.

The solution was adjusted to pH 7.5-8.0 with HCI and placed on a reverse phase (C-18 silica) column 2.5 x 30 cm. The mixture was resolved using a linear gradient of 40-80% methanol in 0.01 M KP0₄ buffer pH 6.85 (1 liter total volume). The individual components were collected as they came off the column in the order di-,8-alanyl hematoporphyrin IX, mono-β-alanyl hematoporphyrin IX and hematoporphyrin IX.

The methanol was removed from each component by flash evaporation and the material was precipitated by adjusting the pH to 2.5 - 3.0 using HCI. The precipitate was washed three times with dilute acetic acid in water at the centrifuge and the products dried under vacuum. The yield of di-β-alanyl hematoporphyrin IX was 52mg and of mono-β-alanyl hematoporphyrin was 30 mg.

### EXAMPLE 7

### Di-L- C( -Serinyl chlcrin e₆ (mixed anhydride method)

650 mg of chlorin e₆ were dissolved in 30 ml of dimethylformide (DMF). 227 µl (0.002 moles) of triethylamine were added to the DMF solution. After stirring for five minutes, 201 µl (0.002 moles) of ethyl chloroformate were added and stirring was continued for an additional 30 minutes. 0.95 g (0.009 moles) of L-a-serine were added to the DMF solution and allowed to stir for one hour at 50-60 C.

The DMF solution was checked for product formation by reverse phase (C-18 silica) TLC using methanol/0.01 M sodium phosphate buffer, pH 6.85, (7.0/3.0) to develop the chromatogram. The DMF solution was flash evaporated to near dryness and the reaction mixture was then taken up in dilute NaOH and the pH was adjusted to 2.5-3.0 to precipitate out the mixture. The precipitate was then centrifuged down and washed twice with diluted acetic acid in water. The precipitate was then centrifuged down and washed twice with diluted acetic acid in water. The precipitate was then redissolved in dilute NaOH and the pH adjusted to 7.0. This was applied to a reverse phase (C-18 silica) column 3.7 cm x 45 cm.

The product was eluted from the column with a solution of 0.01.M sodium phosphate buffer, pH 6.85/methanol (7.0/3.0). Fractions were collected and the fractions of pure di-L-a-serinyl chlorin e₆ were pooled. The methanol was flashed off and the product was precipitated at pH 2.5-3.0. The precipitate was centrifuged down, washed three times with dilute acetic acid, and dried under vacuum. The yield was 200 mg of di-L-a-serinyl chlorin e₆.

Utilizing the aforementioned carbodiimide or the mixed anhydride methods, the following preferred compounds of this invention can be synthesized:

### Chlorin Derivatives

Di - (DL)-serinyl-trans-mesochlorin IX
Di - glycyl-trans-mesochlorin IX
Di -a-(DL)-alanyl-trans-mesochlorin IX
Di -,8-alanyl-trans-mesochlorin IX
Di -_{E}-amino-n-caproyl-mesochlorin IX
Di, tri- (D,L)-serinyl chlorin e₆
Di, tri- (D,L)-serinyl mesochlorin e₆
Di, tri- glycyl chlorin e₆
Di, tri- glycyl mesochlorin e₆
Di, tri-a-(D,L)-alanyl chlorin e₆
Di, tri-a-(D,L)-alanyl mesochlorin e₆
Di, tri-β-alanyl chlorin e₆
Di, tri-β-alanyl mesochlorin e₆
Di, tri-E-amino-n-caproyl chlorin e₆
Di, tri-E-amino-n-caproyl mesochlorin e₆
Di- (D,L)-serinyl chlorin e₄
Di- (D,L)-serinyl mesochlorin e₄
Di- (D,L)-serinyl isochlorin e₄
Di- (D,L)-serinyl mesoisochlorin e₄
Di- glycyl chlorin e₄
Di- glycyl mesochlorin e₄
Di- glycyl isochlorin e₄
Di- glycyl mesoisochlorin e₄
Di-a-(DL)-alanyl chlorin e₄
Di-a-(DL)-alanyl mesochlorin e₄
Di-a-(DL)-alanyl isochlorin e₄
Di-a-(DL)-alanyl mesoisochlorin e₄
Di-β-alanyl chlorin e₄
Di-β-alanyl mesochlorin e₄
Di-β-alanyl isochlorin e₄
Di-β-alanyl mesoisochlorin e₄
Di-_{E}-amino-n-caproyl chlorin e₄
Di-_{E}-amino-n-caproyl mesochlorin e₄
Di-_{E}-amino-n-caproyl isochlorin e₄
Di-_{E}-amino-n-caproyl mesoisochlorin e₄
Di- (D,L)-serinylphotoprotoporphyrin IX
Di- glycylphotoprotoporphyrin IX
Di-a-(D,L)-alanylphotoprotoporphyrin IX
Di-β-alanylphotoprotoporphyrin IX
Di-_{E}-amino-n-caproylphotoprotoporphyrin IX

### Porphyrin Derivatives

Di- (D,L)-serinylmesoporphyrin IX
Di- glycylmesoporphyrin IX
Di-a-(DL)-alanylmesoporphyrin IX
Di-β-alanylmesoporphyrin IX
Di-_{E}-amino-n-caproylmesoporphyrin IX
Di-(D,L)-serinylprotoporphyrin IX
Di-glycylprotoporphyrin IX
Di-a-(D,L)-alanylprotoporphyrin IX
Di-β-alanylprotoporphyrin IX
Di-_{E}-amino-n-caproylprotoporphyrin IX
Di-(D,L)-serinyldeuteroporphyrin IX
Di-glycyldeuteroporphyrin IX
Di-a-(D,L)-alanyldeuteroporphyrin IX
Di-β-alanyldeuteroporphyrin IX
Di-_{E}-amino-n-caproyldeuteroporphyrin IX
Di, tri, tetra- (D,L)-serinylcoproporphyin III
Di, tri, tetra- glycylcoproporphyrin III
ui, tri, tetra-α-(D,L)-alanylcoproporphyrin III
Di, tri, tetra-,8-alanylcoproporphyrin III
Di, tri, tetra-E-amino-n-caproylcoproporphyrin III
Di-(D,L)-serinylhematoporphyrin IX
Di-glycylhematoporphyrin IX
Di-a-(D,L)-alanylhematoporphyrin IX
Di-β-alanylhematoporphyrin IX
Di-_{E}-amino-n-caproylhematoporphyrin IX

### Bacteriochlorin Derivatives

Di-(D,L)-serinylbacteriochlorin e₄
Di-glycylbacteriochlorin e₄
Di-a-(DL)-alanylbacteriochlorin e₄
Di-β-alanylbacteriochlorin e₄
Di-_{E}-amino-n-caproylbacteriochlorin e₄
Di-(D,L)-serinylbacterioisochlorin e₄
Di-glycylbacterioisochlorin e₄
Di-a-(D,L)-alanylbacterioisochlorin e₄
Di-β-alanylbacterioisochlorin e₄
Di-_{E}-amino-n-caproylbacterioisochlorin e₄
Di, tri- (D,L)-serinylbacteriochlorin e₆
Di, tri- glycylbacteriochlorin e₆
Di, tri-a-(D,L)-alanylbacteriochlorin e₆
Di, tri-β-alanylbacteriochlorin e₆
Di, tri-E-amino-n-caproylbacteriochlorin e₆

Similarly, by utilizing other amino acids, peptides which further illustrate embodiments of, but do not limit the present invention, can be employed:
Di-threoninyl trans-mesochlorin IX
Di,tri-threoninyl chlorin e₆
Di,tri-threoninyl mesochlorin e₆
Di-threoninyl chlorin e₄
Di-threoninyl mesochlorin e₄
Di-threoninyl isochlorin e₄
Di-threoninyl mesoisochlorin e₄
Di-threoninyl photoprotoporphyrin IX
Di-threoninyl mesoporphyrin IX
Di-threoninyl protoporphyrin IX
Di-threoninyl deuteroporphyrin IX
Di,tri,tetra-threoninyl coproporphyrin III
Di-threoninyl hematoporphyrin IX
Di-threoninyl bacteriochlorin e₄
Di-threoninyl bacterioisochlorin e₄
Di,tri-threoninyl bacteriochlorin e₆
Di-cysteinyl trans-mesochlorin IX
Di,tri-cysteinyl chlorin e₆
Di,tri-cysteinyl mesochlorin e₆
Di-cysteinyl chlorin e₄
Di-cysteinyl isochlorin e₄
Di-cysteinyl mesoisochlorin e₄
Di-cysteinyl photoprotoporphyrin IX
Di-cysteinyl mesoporphyrin IX
Di-cysteinyl protoporphyrin IX
Di-cysteinyl deuteroporphyrin IX
Di,tri,tetra-cysteinyl-coproporphyrin III
Di-cysteinyl hematoporphyrin IX
Di-cysteinyl bacteriochlorin e₄
Di-cysteinyl bacterioisochlorin e₄
Di, tri-cysteinyl bacteriochlorin e₆
Di-tyrosyl trans-mesochlorin IX
Di, tri-tyrosyl chlorin e₆
Di,tri-tyrosyl mesochlorin e₆
Di-tyrosyl chlorin e₄
Di-tyrosyl mesochlorin e₄
Di-tyrosyl isochlorin e₄
Di-tyrosyl mesoisochlorin e₄
Di-tyrosyl photoprotoporphryin IX
Di-tyrosyl mesoporphyrin IX
Di-tyrosyl protoporphyrin IX
Di-tyrosyl deuteroporphyrin IX
Di,tri,tetra-tyrosyl coproporphyrin III
Di-tyrosyl hematoporphryin IX
Di-tyrosyl bacteriochlorin e₄
Di-tyrosyl bacterioisochlorin e₄
Di,tri-tyrosyl bacteriochlorin e₆
Di-valyl trans-mesochlorin IX
Di,tri-valyl chlorin e₆
Di,tri-valyl mesochlorin e₆
Di-valyl chlorin e₄
Di-valyl mesochlorin e₄
Di-valyl isochlorin e₄
Di-valyl mesoisochlorin e₄
Di-valyl photoprotoporphyrin IX
Di-valyl mesoporphyrin IX
Di-valyl protoporphyrin IX
Di-valyl deuteroporphyrin IX
Di,tri,tetra-valyl coproporphyrin III
Di-valyl hematoporphyrin IX
Di-valyl bacteriochlorin e₄
Di-valyl bacterioisochlorin e₄
Di,tri-valyl bacteriochlorin e₆
Di-leucyl trans-mesochlorin IX
Di,tri-leucyl chlorin e₆
Di,tri-leucyl mesochlorin e₆
Di-leucyl chlorin e₄
Di-leucyl mesochlorin e₄
Di-leucyl isochlorin e₄
Di-leucyl mesoisochlorin e₄
Di-leucyl photoprotoporphyrin IX
Di-leucyl mesoporphyrin IX
Di-leucyl protoporphyrin IX
Di-leucyl deuteroporphyrin IX
Di,tri,tetra-leucyl coproporphyrin III
Di-leucyl hematoporphyrin IX
Di-leucyl bacteriochlorin e₄
Di-leucyl bacterioisochlorin e₄
Di,tri-leucyl bacteriochlorin e₆
Di-isoleucyl trans-mesochlorin IX
Di,tri-isoleucyl chlorin e₆
Di,tri-isoleucyl mesochlorin e₆
Di-isoleucyl chlorin e₄
Di-isoleucyl mesochlorin e₄
Di-isoleucyl isochlorin e₄
Di-isoleucyl mesoisochlorin e₄
Di-isoleucyl photoprotoporphyrin IX
Di-isoleucyl mesoporphyrin IX
ui-isoleucyl protoporphyrin IX
Di-isoleucyl deuteroporphyrin IX
Di,tri,tetra-isoleucyl coproporphyrin III
Di-isoleucyl hematoporphyrin IX
Di-isoleucyl bacteriochlorin e₄
Di-isoleucyl bacterioisochlorin e₄
Di,tri-isoleucyl bacteriochlorin e₆
Di-prolyl trans-mesochlorin IX
Di,tri-prolyl chlorin e₆
Di,tri-prolyl mesochlorin e₆
Di-prolyl chlorin e₄
Di-prolyl mesochlorin e₄
Di-prolyl isochlorin e₄
Di-prolyl mesoisochlorin e₄
Di-prolyl photoprotoporphyrin IX
Di-prolyl mesoporphryin IX
Di-prolyl protoporphyrin IX
Di-prolyl deuteroporphyrin IX
Di,tri,tetra-prolyl coproporphyrin III
Di-prolyl hematoporphyrin IX
Di-prolyl bacteriochlorin e₄
Di-prolyl bacteriiosochlorin e₄
Di,tri-prolyl bacteriochlorin e₆
Di-phenylalanyl trans-mesochlorin IX
Di,tri-phenylalanyl chlorin e₆
Di,tri-phenylalanyl mesochlorin e₆
Di-phenylalanyl chlorin e₄
Di-phenylalanyl mesochlorin e₄
Di-phenylalanyl isochlorin e₄
Di-phenylalanyl mesoisochlorin e₄
Di-phenylalanyl photoprotoporphyrin IX
Di-phenylalanyl mesoporphyrin IX
Di-phenylalanyl protoporphyrin IX
Di-phenylalanyl deuteroporphyrin IX
Di,tri,tetra-phenylalanyl coproporphyrin III
Di-phenylalanyl hematoporphyrin IX
Di-phenylalanyl bacteriochlorin e₄
Di-phenylalanyl bacterioisochlorin e₄
Di,tri-phenylalanyl bacteriochlorin e₆
Di-tryptophyl trans-mesochlorin IX
Di,tri-tryptophyl chlorin e₆
Di,tri-tryptophyl mesochlorin e₆
Di-tryptophyl chlorin e₄
Di-tryptophyl mesochlorin e₄
Di-tryptophyl isochlorin e₄
Di-tryptophyl mesoisochlorin e₄
Di-tryptophyl photoprotoporphyrin IX
Di-tryptophyl mesoporphyrin IX
Di-tryptophyl protoporphyrin IX
Di-tryptophyl deuteroporphyrin IX
Di,tri,tetra-tryptophyl coproporphyrin III
Di-tryptophyl hematoporphyrin IX
Di-tryptophyl bacteriochlorin e₄
Di-tryptophyl bacterioisochlorin e₄
Di,tri-tryptophyl bacteriochlorin e₆
Di-methionyl trans-mesochlorin IX
Di,tri-methionyl chlorin e₆
Di,tri-methionyl mesochlorin e₆
Di-methionyl chlorin e₄
Di-methionyl mesochlorin e₄
Di-methionyl isochlorin e₄
Di-methionyl mesoisochlorin e₄
Di-methionyl photoprotoporphyrin IX
Di-methionyl mesoporphyrin IX
Di-methionyl protoporphyrin IX
Di-methionyl deuteroporphyrin IX
Di,tri,tetra-methionyl coproporphyrin III
Di-methionyl hematoporphyrin IX
Di-methionyl bacteriochlorin e₄
Di-methionyl bacterioisochlorin e₄
Di,tri-methionyl bacteriochlorin e₆
Di-histidyl trans-mesochlorin IX
Di,tri-histidyl Chlorin e₆
Di,tri-histidyl mesochlorin e₆
Di-histidyl chlorin e₄
Di-histidyl mesochlorin e₄
Di-histidyl isochlorin e₄
Di-histidyl mesoisochlorin e₄
Di-histidyl photoprotoporphyrin IX
Di-histidyl mesoporphyrin IX
Di-histidyl protoporphyrin IX
Di-histidyl deuteroporphyrin IX
Di tri,tetra-histidyl coproporphyrin III
Di-histidyl hematoporphyrin IX
Di-histidyl bacteriochlorin e₄
Di-histidyl bacterioisochlorin e₄
Di,tri-histidyl bacteriochlorin e₆
Di-arginyl trans-mesochlorin IX
Di,tri-arginyl chlorin e₆
Di,tri-arginyl mesochlorin e₆
Di-arginyl chlorin e₄
Di-arginyl mesochlorin e₄
Di-arginyl isochlorin e₄
Di-arginyl mesoisochlorin e₄
Di-arginyl photoprotoporphyrin IX
Di-arginyl mesoporphryin IX
Di-arginyl protoporphyrin IX
Di-arginyl deuteroporphyrin IX
Di,tri,tetra-arginyl coproporphyrin III
Di-arginyl hematoporphyrin IX
Di-arginyl bacteriochlorin e₄
Di-arginyl bacterioisochlorin e₄
Di,tri-arginyl bacteriochlorin e₆
Di-lysyl trans-mesochlorin IX
Di,tri-lysyl chlorin e₆
Di,tri-lysyl mesochlorin e₆
Di-lysyl chlorin e₄
Di-lysyl mesochlorin e₄
Di-lysyl isochlorin e₄
Di-lysyl mesoisochlorin e₄
Di-lysyl photoprotoporphyrin IX
Di-lysyl mesoporphyrin IX
Di-lysyl protoporphyrin IX
Di-lysyl deuteroporphyrin IX
Di,tri,tetra-lysyl coproporphyrin III
Di-lysyl hematoporphyrin IX
Ui-lysyl bacteriochlorin e₄
Di-lysyl bacterioisochlorin e₄
Di,tri-lysyl bacteriochlorin e₆
Di-glutaminyl trans-mesochlorin IX
Di,tri-glutaminyl chlorin e₆
Di,tri-glutaminyl mesochlorin e₆
Di-glutaminyl chlorin e₄
Di-glutaminyl mesochlorin e₄
Di-glutaminyl isochlorin e₄
Di-glutaminyl mesoisochlorin e₄
Di-glutaminyl photoprotoporphyrin IX
Di-glutaminyl mesoporphyrin IX
Di-glutaminyl protoporphyrin IX
Di-glutaminyl deuteroporphyrin IX
Di,tri,tetra-glutaminyl coproporphyrin III
Di-glutaminyl hematoporphyrin IX
Di-glutaminyl bacteriochlorin e₄
Di-glutaminyl bacterioisochlorin e₄
Di,tri-glutaminyl bacteriochlorin e₆
Di-asparginyl trans-mesochlorin IX
Di,tri-asparginyl chlorin e₆
Di,tri-asparginyl mesochlorin e₆
Di-asparginyl chlorin e₄
Di-asparginyl mesochlorin e₄
Di-asparginyl isochlorin e₄
Di-asparginyl mesoisochlorin e₄
Di-asparginyl photoprotoporphyrin IX
Di-asparginyl mesoporphyrin IX
Di-asparginyl protoporphyrin IX
Di-asparginyl deuteroporphyrin IX
Di,tri,tetra-asparginyl coproporphyrin III
Di-asparginyl hematoporphyrin IX
Di-asparginyl bacteriochlorin e₄
Di-asparginyl bacterioisochlorin e₄
Di,tri-asparginyl bacteriochlorin e₆

### Monoamides

### EXAMPLE 8

### Mono (DL) serinyl mesoporphyrin IX (mixed anhydride method)

400 mg (0.0007 moles) of mesoporphyrin IX were suspended in 50 ml of tetrahydrofuran (THF). 360 µl (0.0035 moles) of triethylamine were added with stirring. After 10 minutes, 340 µl (0.0031 moles) ethyl chloroformate were added. After stirring 10 minutes, 10 ml (0.01 moles) of 1 M KOH containing 761 mg (0.0072 moles) of DL serine were added to the THF solution. This mixture was stirred 60 minutes at room temperature.

The organic solvent was flashed off and the reaction mixture was checked by silica TLC for product. Benzene/methanol/88% formic acid (8.5/1.5/0.13) was used to develop the chromatrogram.

After checking for product, the solution was adjusted to pH 7.5-8.0 and placed on a reverse phase (C-18 silica) column 2.5 x 30 cm. The reaction mixture was resolved using a linear gradient of 20-70% methanol in 0.01 M KP0₄ buffer pH 6.85 (1 liter total volume).

The column effluent was collected via fraction collector and the tube contents were pooled according to individual components. The order of elution was diserinyl mesoporphyrin IX, monoserinyl mesoporphyrin IX and unsubstituted mesoporphyrin IX.

The methanol was flashed off and the material was precipitated at pH 2.5-3.0. The ppt. was washed 3 times with dilute acetic acid in water. The product was dried under vacuum.

### Example 9

### Mono glycyl mesoporphyrin IX (mixed anhydride method)

100 mg (0.000175 moles) of mesoporphyrin IX were suspended in 100 ml of tetrahydrofuran (THF). 360 µl (0.0035 moles) of triethylamine were added with stirring. After 10 minutes, 340 µl (0.0031 moles) of ethylchloroformate were added. After stirring 10 minutes, 10 ml (0.01 moles) of 1 M KOH containing 500 mg (0.0066 moles) of glycine were added to the THF solution. This mixture was stirred 60 minutes at room temperature.

The organic solvent was flashed off and the reaction mixture was checked silica TLC for product. Benzene/methanol/88% formic acid (8.5/1.5/0/13) was used to develop the chromatogram.

After checking for product, the solution was adjusted to pH 7.5-8.0 and placed on a reverse phase (C-18 silica) column 2.5 x 30 cm. The reaction mixture was resolved using a linear gradient of zero to 50% methanol in 0.01 M KP0₄ buffer pH 6.85 (1 total volume).

The column effluent was collected in a fraction co-lector and the contents were sorted according to individual components. The order of elution was diglycyl mesoporphyrin IX, monoglycyl mesoporphyrin IX and unsubstituted mesoporphyrin IX.

The methanol was flashed off and the material was precipitated at pH 2.5-3.0. The ppt. was washed 3 times with dilute acetic acid in water. The product was dried under vacuum.

### EXAMPLE 10

### Mono a (DL) alanyl mesoporphyrin IX (Mixed anhydride method)

100 mg (0.000175 moles) of mesoporphyrin IX were suspended in 100 ml of tetrahydrofuran (THF). 210 µl (0.002 moles) of triethylamine were added with stirring. After 10 minutes 195 µl (0.00177 moles) of ethylchloroformate were added. After stirring 10 minutes, 10 ml (0.01 moles) of 1 M KOH containing 500 mg (0.0056 moles) of a(DL) alanine were added to the THF solution. This mixture was stirred 60 minutes at room temperature.

The organic solvent was flashed off and the reaction mixture was checked by silica TLC for product. Benzene/methanol/88% formic acid (8.5/1.5/0.13) was used to develop the chromatogram.

After checking for product, the solution was adjusted to pH 7.5-8.0 and placed on a reverse phase (C-18 silica) column 2.5 x 30 cm. The reaction mixture was resolved using a linear gradient of 20-70% methanol in 0.01 M KP0₄ buffer pH 6.85 (1 R total volume).

The column effluent was collected via fraction collection and the tube contents were sorted according to individual components. The order of elution was di-a(DL) alanyl mesoporphyrin IX, mono- a(DL)-alanyl mesoporphyrin IX and unsubstituted mesoporphyrin IX.

The methanol was flashed off and the material was precipitated at pH 2.5-3.0. The ppt. was washed 3 times with dilute acetic acid in water. The product was dried under vacuum.

### EXAMPLE 11

### Mono alanyl mesoporphyrin IX (mixed anhydride method)

400 mg (0.0007 moles) of mesoporphyrin IX were suspended in 100 ml of tetrahydrafuran (THF). 360 µl (0.0035 moles) of triethylamine were added with stirring. After 10 minutes, 340 µl (0.0031 moles) ethyl chloroformate were added. After stirring 10 minutes, 10 ml (0.01) moles) of 1 M KOH containing 400 mg (0.0044 moles) of alanine were added to the THF solution. This mixture was stirred 60 minutes at room temperature.

The organic solvent was flashed off and the reaction mixture was checked by silica TLC for product. Benzene/methanol/88% formic acid (8.5/1,5/0.13) was used to develop the chromatogram.

After checking for product, the solution was adjusted to pH 7.5-8.0 and placed on a reverse phase (C-18 silica) column 2.5 x 30 cm. The reaction mixture was resolved using a linear gradient of 40-80% methanol in 0.01 M KP0₄ buffer pH 6.85 (1 R total volume).

The column effluent was collected via fraction collector and the tube contents were pooled according to individual components. The order of elution was di-β-alanyl mesoporphyrin IX, mono-β-alanyl mesoporphyrin IX, and unsubstituted mesoporphyrin IX.

The methanol was flashed off and the material was precipitated at pH 2.5-3.0. The ppt. was washed 3 times with dilute acetic acid in water. I he product was dried under vacuum. I he yield tor mono-β-alanyl mesoporphyrin IX was 23 mg.

### EXAMPLE 12

### Mono _{E} amino-n-caproyl mesoporphyrin IX (mixed anhydride method,

400mg (0.0007 moles) of mesoporphyrin IX were suspended in 50 ml of tetrahydrofuran (THF). 360 µl (0.0035 moles) of triethylamine were added with stirring. After 10 minutes, 340 µl (0.00414 moles) of ethylchloroformate were added. After stirring 10 minutes, 10 ml (0.01 moles) of 1 M KOH containing 543 mg (0.00369 moles) of e-amino-n-caproic acid were added to the THF solution. This mixture was stirred 60 minutes at room temperature.

The organic solvent was flashed off and the reaction mixture was checked by silica TLC for product. Benzene/methanol/88% formic acid (8.5/1.5/0.13) was used to develop the chromatogram.

After checking for product, the solution was adjusted to pH 7.5-8.0 and placed on a reverse phase (C-18 silica) column 2.5x30cm. The reaction mixture was resolved using a linear gradient of 20-70% methanol in 0.01 M KP0₄ buffer pH 6.85 (1 R total volume).

The column effluent was collected via fraction collector and the tube contents were pooled according to individual components. The order of elution was di-_{E}-amino-n-caproyl mesoporphyrin IX, mono-_{E}-amino-n-caproyl mesoporphyrin IX, and unsubstituted mesoporphyrin IX.

The methanol was flashed off and the material was precipitated at pH 2.5-3.0. The ppt. was washed three times with dilute acetic acid in water. The product was dried under vacuum.

### EXAMPLE 13

### Mono -β-alanyl hematoporphyrin IX (mixed anydride method)

400 mg (0.00059 moles) of hematoporphyrin IX dihydrochloride were suspended in 50 ml of tetrahydrofuran (THF).

360 µl (0.0035 moles) of triethylamine were added with stirring. After 10 minutes, 340 µl (0.0031 moles) of ethyl chloroformate were added. After stirring 10 minutes, 10 ml (0.01 moles) of 1 M KOH containing 400 mg (0.0044 moles) of β-alanine were added to the THF solution. This mixture gas stirred 60 minutes at room temperature.

The organic solvent was removed by flash evaporation, keeping the temperature below 50 ° C. The reaction mixture was checked for product by silica TLC using Benzene/methanol 88% formic acid (8.5/1.5/0.13) as solvent to develop the chromatogram.

The solution was adjusted to pH 7.5-8.0 with HCI and placed on reverse phase (C-18 silica) column 2.5 x 30 cm. The mixture was resolved using a linear gradient of 40-80% methanol in 0.01 M KP0₄ buffer pH 6.65 (1 liter total volume). The individual components were collected as they came off the column in the order di-,8 alanyl hematoporphyrin IX, mono -β- alanyl hematoporphyrin IX and hematoporphyrin IX.

The methanol was removed from each component by flash evaporation and the material was precipitated by adjusting the pH to 2.5 -3.0 using HCI. The precipitate was washed three times with dilute acetic acid in water at the centrifuge and the products dried under vacuum. The yield of di-β- alanyl hematoporphyrin IX was 52mg and of mono - β-alanyl hematoporphyrin was 30 mg.

### EXAMPLE 14

### Mono glycyl chlorin e₆ (Mixed Anhydride Method)

625 mg of chlorin e₆ were dissolved in 300 ml of dimethyl formamide (DMF) and 277 µl (0.002 moles) of triethylamine (TEA) were added to the DMF solution. After stirring for five minutes, 201 µl (0.002 moles) of ethylchloroformate (EC) were added and stirred for 1 1/2 hours at room temperature.

75 mg (.0009 moles) of glycine (ammonia free) were added to the DMF solution and allowed to stir three hours at 50-60 ° C.

The DMF solution was tested for product by reverse phase (C-18 silica) TLC using methanol/0.01 M sodium phosphate buffer, pH 6.85, 70/30, to develop the chromatogram.

The DMF solution was flashed to near dryness, then dissolved in dilute NaOH and the pH adjusted to 2.5-3 to precipitate the solid. The precipitate was then placed on a reverse phase (C-18 silica) column 3.7 cm x 45 cm.

Fractions were eluted, using 20-40% methanol in 0.01 M sodium phosphate buffer, pH 6.85. The fractions were pooled according to individual components.

The methanol was flashed off and the material was precipitated at pH 2.5-3.0. The precipitate was washed and centrifuged 3 times in dilute acetic acid in water. The product was dried under vacuum. The yield of mono glycyl chlorin e₆ was 87.5 mg.

### EXAMPLE XV

### Preparation of mono-L-serinyl chlorin e₆

Chlorin e₆ was prepared according to the procedure of Fischer and Stern, Die Chemie Des Pyrroles, Volume II, second half, Leipzig 1940, Akademische Verlagsgesellschaft, pp. 91-93.

100 mg of the chlorin e₆ (free acid form) and 35 mg of 1-ethyl-3-(3-dimethylaminopropyl) carbodiimide hydrochloride were dissolved in 2 ml of N, N'-dimethyl formamide. After 5 minutes, 125 mg of L-serine benzyl ester hydrochloride was added, stirred vigorously until solution was complete, then allowed to stand at room temperature for 2 hours. At this time 0.5 ml of glacial acetic acid were added, then 30 ml of methanol and 12 ml of H₂0.

The solution was applied to a C-18 reverse phase column (14 x 2 cm). The column was washed with H₂0 (100 ml) then 4 ml of 1 M NH₄0H, then with H₂0 again (50 ml). Eluted product with MeOH/H₂0. Fractions eluted from the column with 30% to 80% MeOH contained product as well as carbodiimide activated chlorin as determined by TLC on C-18 reverse phase plates with solvent 70% MeOH/30% buffer (0.1 M sodium phosphate pH 6.85) V/V.

These fractions were pooled and enough 3 N NaOH was added to make the solution 0.1 N in NaOH. After 1 hour, the hydrolysis was complete as determined by TLC in the above system. Removed the methanol by rotary evaporation and adjusted the pH of the solution to 7.5 with HCI. The chlorin solution was then reapplied to the same reverse phase column, washed with water, and eluted with MeOH/water using a stepwise gradient from 10 to 50% methanol. The fractions containing pure mono-L-serinyl chlorin as determined by TLC (R_{f} slightly greater than the unsubstituted chlorin) were pooled, the methanol removed by rotary evaporation, and the product dried as the trisodium salt by lyophylization.

### EXAMPLE XVI

### Preparation of mono-L-asparaginyl chlorin e₆

500 mg of chlorin e₆ and 175 mg of 1-ethyl-3-(3-dimethylamine-propyl) carbodiimide hydrochloride were dissolved in 10 ml of N, N'-dimethyl formamide. After 5 minutes, 410 mg of L-asparagine were added. The solution was agitated for 4 hours. The asparagine did not dissolve totally during this reaction, but reverse phase (C-18) TLC 70/30 MeOH/.01 M sodium phosphate buffer pH 6.85 showed some product at this time, (R_{f} slightly greater than chlorin e₆). The reaction was terminated by adding 2.5 ml glacial acetic acid, then diluting to a total volume of 100 ml with methanol, then adding 25 ml of H₂0 slowly, with stirring. The solution was then applied to a 14 x 2 cm reverse phase (C-18) column, washed with water then with 5 ml of 0.1 M NaOH, finally with 50 ml of 0.01 M sodium phosphate buffer, pH 6.85. The product was eluted off with MeOH/H₂0 in a stepwise gradient from 20% MeOH to 50% MeOH. The fractions containing pure mono-L-asparaginyl chlorin e₆, as determined by TLC using the conditions stated above, were pooled, and the methanol removed by rotary evaporation. The product was isolated as the trisodium salt by lyoph- lization.

### EXAMPLE XVII

### Preparation of mono-L-cysteinyl chlorin e₆

300 mg of chlorin e₆ and 105 mg of 1-ethyl-3-(3-dimethylamine-propyl)carbodiimide hydrochloride were dissolved in 6 ml of N, N'-dimethylformamide. After 5 minutes, 255 mg of L-cysteine hydrochloride were added. The solution was stirred at room temperature for 5 hours. The rest of the procedure is the same as for the preparation of mono-L-asparaginyl chlorin e₆.

### EXAMPLE XVIII

### Preparation of mono-L-serinyl-2- formylchlorin e₆ (mono-L-serinyl 2-desvinyl-2-formyl-chlorin e₆)

500 mg of chlorin e₆ trimethyl ester were prepared according to the procedure of Fischer and Stern, in Die Chemie Des Pyrroles, Volume II, second half, Leipzig 1940, Akademische Verlagsgesellschaft, pp. 98-102. The chlorin e₆ trimethyl ester was dissoved in 600 ml of refluxing acetone. 400 mg of Potassium permanganate and 800 mg of magnesium sulfate dissolved in 130 ml of H₂0 were added slowly over approximately a one hour period to the reluxing acetone solution. The solution was allowed to reflux for 1/2 hour after addition was complete. After cooling, 300 ml of methylene chloride were added, and the mixture was washed 3 times with water in a separatory funnel. The volume of methylene chloride was reduced and the product chromatographed on silica gel, eluting with a gradually increasing percentage of ethyl acetate in the CH₂CI₂. The first major brown band which eluted was collected as the product, 2-Desvinyl-2-FormylChlorin e₆. Yield 94 mg.

The product was saponified by dissolution in refluxing n-propanol (0.1 ml/mg) and addition of 6 fold equivalent of 1 N KOH. The tripotassium salt was filtered off, washed with n-propanol and dried under vacuum, forming 2-formyl chlorin e₆.

100 mg of the 2-formyl chlorin e₆ free acid form) and 35 mg of 1-ethyl-3-(3-dimethylaminopropyl) carbodiimide hydrochloride were dissolved in 2 ml of N, N'-dimethyl formamide. After 5 minutes, 125 mg of L-serine benzyl ester hydrochloride was added, stirred vigorously until solution was complete, then allowed to stand at room stemperature for 2 hours. At this time 0.5 ml of glacial acetic acid was added, then 30 ml of methanol and 12 ml of H₂0.

The solution was applied to a C-18 reverse phase column (14 x 2 cm). The column was washed with H₂0 (100 ml) then 4 ml of 1 M NH₄0H, then with H₂0 again (50 ml). Eluted product with MeOH/H₂0. Fractions eluted from the column with 30% to 80% MeOH contained product as well as carbodiimide activated chlorin as determined by TLC on C-18 reverse phase plates with solvent 70% MeOH/30% buffer (0.1 M sodium phosphate pH 6.85) V/V.

These fractions were pooled and enough 3 N NaOH was added to make the solution 0.1 N in NaOH. After 1 hour, the hydrolysis was complete as determined by TLC in the above system. Removed the methanol by rotary evaporation and adjusted the pH of the solution to 7.5 with HCI. The chlorin solution was then reapplied to the same reverse phase column, washed with water, and eluted with MeOH/water using a stepwise gradient from 10 to 50% methanol. The fractions containing pure mono-L-serinyl chlorin as determined by TLC (R_{f} slightly greater than the unsubstituted chlorin) were pooled, the methanol removed by rotary evaporation, and the product dried as the trisodium salt by lyophylization.

### EXAMPLE XIX

### Preparation of mono-L-serinyl-deuterochlorin e6 (Mono-L-Serinyl 2-desvinyl-chlorin e₆)

### A. Deuterochlorin e₆

Deuterchlorin e₆ trimethyl ester was prepared according to the procedure in Fischer and Stern in Die Chemie Des Pyrroles, Volume II, second half, Leipzig 1940, Akademische Verlagsgesellschaft, p. 104. The trimethyl ester was then hydrolyzed to the free acid state by dissolution in refluxing n-propanol (0.1 ml/mg) and adding 6 fold equivalent amounts of 1 N KOH. The product was collected by filtration, after cooling, as the potassium salt and dried under vaccum.

### B. Mono-L-Serinyl Deuterochlorin e₆

100 mg of the deuterchlorin e₆ (free acid form) and 35 mg of 1-ethyl-3-(3-dimethylaminopropyl) carbodiimide hydrochloride were dissolved in 2 ml of N, N' dimethyl formamide. After 5 minutes, 125 mg of L-serine benzyl ester hydrochloride were added, stirred vigorously until solution was complete, then allowed to stand at room temperature for 2 hours. At this time 0.5 ml of glacial acetic acid were added, then 30 ml of methanol and 12 ml of H₂0.

The solution was applied to a C-18 reverse phase column (14 x 2 cm). The column was washed with H₂0 (100 ml) then 4 ml of 1 M NH₄0H, then with H₂0 again (50 ml). Eluted product with MeOH/H₂0. Fractions eluted from the column with 30% to 80% MeOH contained product as well as carbodiimide activated chlorin as determined by TLC on C-18 reverse phase plates with solvent 70% MeOH/30% buffer (0.1 M sodium phosphate pH 6.85) V/V.

These fractions were pooled and enough 3 N NaOH was added to make the solution 0.1 N in NaOH. After 1 hour, the hydrolysis was complete as determined by TLC in the above system. Removed the methanol by rotary evaporation and adjusted the pH of the solution to 7.5 with HCI. The chlorin solution was then reapplied to the same reverse phase column, washed with water, and eluted with MeOH/water using a stepwise gradient from 10 to 50% methanol. The fractions containing pure mono-L-serinyl chlorin, as determined by TLC (R_{f} slightly greater than the unsubstituted chlorin) were pooled, the methanol removed by rotary evaporation, and the product dried as the trisodium salt by lyophylization.

### EXAMPLE XX

### Preparation of mono-L-serinyl-2 acetyl-chlorin e₆ (Mono-L-serinyl-2-desvinyl-2-acetyl chlorin e₆)

### A. 2-acetyl chlorin e₆

2-acetyl chlorin e₆ trimethyl ester was prepared according to the procedure of Fischer and Stern, Die Chemie Des Pyrroles, Volume II, second half, Leipzig 1940, Akademische Verlagsgesellschaft, p. 185. The trimethyl ester was then hydrolyzed to the free acid state by dissolution in refluxing n-propanol (0.1 ml/mg) and adding 6 fold equivalent amounts of 1 N KOH. The product was collected by filtration, after cooling, as the potassium salt and dried under vaccum.

### B. L-serinyl-2-acetyl chlorin e₆

100 mg of the 2-acetyl chlorin e₆ (free acid form) and 35 mg of 1-ethyl-3-(3-dimethylaminopropyl) carbodiimide hydrochloride were dissolved in 2 ml of N, N'-dimethyl formamide. After 5 minutes, 125 mg of L-serine benzyl ester hydrochloride were added, stirred vigorously until solution was complete, then allowed to stand at room temperature for 2 hours. At this time 0.5 ml of glacial acetic acid were added, then 30 ml of methanol and 12 ml of H₂0.

The solution was applied to a C-18 reverse phase column (14 x 2 cm). The column was washed with H₂0 (100 ml) then 4 ml of 1 M NH₄0H, then with H₂0 again (50 ml). Eluted product with MeOH/H₂0. Fractions eluted from the column with 30% to 80% MeOH contained product as well as carbodiimide activated chlorin as determined by TLC on C-18 reverse plates with solvent 70% MeOH/30% buffer (.01 M sodium phosphate, pH 6.85) V/V.

These fractions were pooled and enough 3N NaOH was added to make the solution 0.1 N in NaOH. After 1 hour, the hydrolysis was complete as determined by TLC in the above system. Removed the methanol by rotary evaporation and adjusted the pH of the solution to 7.5 with HCI. The chlorin solution was then reapplied to the same reverse phase column, washed with water, and eluted with MeOH/water using a stepwise gradient from 10 to 50% methanol. The fractions containing pure mono-L-serinyl chlorin as determined by TLC (R_{f} slightly greater than the unsubstituted chlorin) were pooled, the methanol removed by rotary evaporation, and the product dried as the trisodium salt by lyophylization.

### EXAMPLE XXI

### Preparation of mono-L-serinyl mesochlorin e₆

### A. Mesochlorin e₆

Mesochlorin e₆ trimethyl ester was prepared according to the procedure of Fischer and Stern, Die Chemie Des Pyrroles, Volume II, second half, Leipzig 1940, Akademische Verlagsgesellschaft p. 102.

The mesochlorin e₆ trimethyl ester was then hydrolyzed to the free acid state by dissolution in refluxing n-propanol (0.1 ml/mg) and adding 6 fold equivalent amounts of 1 N KOH. The product was collected by filtration, after cooling, as the potassium salt and dried under vacuum.

### B. Mono-L-Serinyl Mesochlorin e₆

100 mg of the mesochlorin e₆ (free acid form) and 35 mg of 1-ethyl-3-(3-dimethylaminopropyl) carbodiimide hydrochloride were dissolved in 2 ml of N, N'-dimethyl formamide. After 5 minutes, 125 mg of L-serine benzyl ester hydrochloride were added, stirred vigorously until solution was complete, then allowed to stand at room temperature for 2 hours. At this time 0.5 ml of glacial acetic acid were added, then 30 ml of methanol and 12 ml of H₂0.

The solution was applied to a C-18 reverse phase column (14 x 2 cm). The column was washed with H₂0 (100 ml) then 4 ml of 1 M NH₄0H, then with H₂0 again (50 ml). Eluted product with MeOH/H₂0. Fractions eluted from the column with 30% to 80% MeOH contained product as well as carbodiimide activated chlorin as determined by TLC on C-18 reverse phase plates with solvent 70% MeOH/30% buffer (.01 M sodium phosphate pH 6.85) V/V.

These fractions were pooled and enough 3 N NaOH was added to make the solution 0.1 N in NaOH. After 1 hour, the hydrolysis was complete as determined by TLC in the above system. Removed the methanol by rotary evaporation and adjusted the pH of the solution to 7.5 with HCI. The chlorin solution was then reapplied to the same reverse phase column, washed with water, and eluted with MeOH/water using a stepwise gradient from 10 to 50% methanol. The fractions containing pure mono-L-serinyl chlorin was determined by TLC (R_{f} slightly greater than the unsubstituted chlorin) were pooled, the methanol removed by rotary evaporation, and the product dried as the trisodium salt by lyophylization.

Utilizing the aforementioned carbodiimide or the mixed anhydride methods of Exampies 8-21 the following preferred monoamide compounds of this invention are synthesized:

### Chlorin Derivatives

(DL)-Serinyl-trans-mesochlorin IX
Glycyl-trans-mesochlorin IX
a-(DL)-Alanyl-trans-mesochlorin IX
β-Alanyl-trans-mesochlorin IX
_{E}-Amino-n-caproyl-mesochlorin IX
(D,L)-Serinyl chlorin e₆
(D,L)-Serinyl mesochlorin e₆
Glycyl chlorin e₆
Glycyl mesochlorin e₆
a-[D,L)-Alanyl chlorin e₆
a-(D,L) Alanyl mesochlorin e₆
β-Alanyl chlorin e₆
β-Alanyl mesochlorin e₆
ε-Amino-n-caproyl chlorin e₆
ε-Amino-n-caproyl mesochlorin e₆
(D,L)-Serinyl chlorin e₄
(D,L)-Serinyl mesochlorin e₄
(D,L)-Serinyl isochlorin e₄
(D,L)-Serinyl mesoisochlorin e₄
Glycyl chlorin e₄
Glycyl mesochlorin e₄
Glycyl isochlorin e₄
Glycyl mesoisochlorin e₄
a-(DL)-Alanyl chlorin e₄
a-(DL)-Alanyl mesochlorin e₄
a-(DL)-Alanyl isochlorin e₄
a-(DL)-Alanyl mesoisochlorin e₄
β-Alanyl chlorin e₄
β-Alanyl mesochlorin e₄
β-Alanyl isochlorin e₄
β-Alanyl mesoisochlorin e₄
ε-Amino-n-caproyl chlorin e₄
ε-Amino-n-caproyl mesochlorin e₄
ε-Amino-n-caproyl isochlorin e₄
ε-Amino-n-caproyl mesoisochlorin e₄
(D,L)-Serinylphotoprotoporphyrin IX
Glycylphotoprotoporphyrin IX
a-(D,L)-Alanylphotoprotoporphyrin IX
β-Alanylphotoprotoporphyrin IX
_{E}-Amino-n-caproylphotoprotoporphyrin IX
Threoninyl chlorin e₆
Tyrosyl chlorin e₆
Valyl chlorin e₆
Leucyl chlorin e₆
lsoleucyl chlorin e₆
Prolyl chlorin e₆
Methionyl chlorin e₆
Histidyl chlorin e₆
Arginyl chlorin e₆
Lysyl chlorin e₆
Glutaminyl chlorin e₆
4-hydroxyprolyl chlorin e₆
5-hydroxylysyl chlorin e₆
ε amino-n-caproyl chlorin e₆
γ aminobutanoyl chlorin e₆
3- methyl histidyl chlorin e₆
Alanyl 2-acetyl-chlorin e₆
Valyl 2-acetyl-chlorin e₆
Leucyl 2-acetyl-chlorin e₆
Isoleucyl 2-acetyl-chlorin e₆
Prolyl 2-acetyl-chlorin e₆
Methionyl 2-acetyl-chlorin e₆
Glycyl 2-acetyl-chlorin e₆
Serinyl 2-acetyl-chlorin e₆
Threoninyl 2-acetyl-chlorin e₆
Cysteinyl 2-acetyl-chlorin e₆
Tyrosyl 2-acetyl-chlorin e₆
Asparginyl 2-acetyl-chlorin e₆
Lysyl 2-acetyl-chlorin e₆
Arginyl 2-acetyl-chlorin e₆
Histidyl 2-acetyl-chlorin e₆
Glutaminyl 2-acetyl-chlorin e₆
4-hydroxy-prolyl 2-acetyl-chlorin e₆
5-hydroxy lysyl 2-acetyl-chlorin e₆
_{E}-amino-n-caproyl 2-acetyl-chlorin e₆
y-aminobutanoyl 2-acetyl-chlorin e₆
3-methyl histidyl 2-acetyl-chlorin e₆
β-alanyl 2-acetyl-chlorin e₆
Alanyl 2 formyl chlorin e₆
Valyl 2 formyl chlorin e₆
Leucyl 2 formyl chlorin e₆
Isoleucyl 2 formyl chlorin e₆
Prolyl 2 formyl chlorin e₆
Methionyl 2 formyl chlorin e₆
Glycyl 2 formyl chlorin e₆
Serinyl 2 formyl chlorin e₆
Threoninyl 2 formyl chlorin e₆
Cysteinyl 2 formyl chlorin e₆
Tyrosyl 2 formyl chlorin e₆
Asparginyl 2 formyl chlorin e₆
Lysyl 2 formyl chlorin e₆
Arginyl 2 formyl chlorin e₆
Histidyl 2 formyl chlorin e₆
Glutaminyl 2 formyl chlorin e₆
4-hydroxy-prolyl 2 formyl chlorin e₆
5-hydroxy lysyl 2 formyl chlorin e₆
ε-amino-n-caproyl 2 formyl chlorin e₆
y-aminobutanoyl 2 formyl chlorin e₆
3-methyl histidyl 2 formyl chlorin e₆
β-alanyl 2 formyl chlorin e₆
Alanyl Deuterochlorin e₆
Valyl Deuterochlorin e₆
Leucyl Deuterochlorin e₆
Isoleucyl Deuterochlorin e₆
Prolyl Deuterochlorin e₆
Methionyl Deuterochlorin e₆
Glycyl Deuterochlorin e₆
Serinyl Deuterochlorin e₆
Threoninyl Deuterochlorin e₆
Cysteinyl Deuterochlorin e₆
Tyrosyl Deuterochlorin e₆
Asparginyl Deuterochlorin e₆
Lysyl Deuterochlorin e₆
Arginyl Deuterochlorin e₆
Histidyl Deuterochlorin e₆
Glutaminyl Deuterochlorin e₆
4-hydroxy-prolyl Deuterochlorin e₆
5-hydroxy lysyl Deuterochlorin e₆
ε-amino-n-caproyl Deuterochlorin e₆
y-aminobutanoyl Deuterochlorin e₆
3-methyl histidyl Deuterochlorin e₆
β-alanyl Deuterochlorin e₆
valyl mesochlorin e₆
Leucyl mesochlorin e₆
Isoleucyl mesochlorin e₆
Prolyl mesochlorin e₆
Methionyl mesochlorin e₆
Serinyl mesochlorin e₆
Threoninyl mesochlorin e₆
Cysteinyl mesochlorin e₆
Tyrosyl mesochlorin e₆
Asparginyl mesochlorin e₆
Lysyl mesochlorin e₆
Arginyl mesochlorin e₆
Histidyl mesochlorin e₆
Glutaminyl mesochlorin e₆
4-hydroxy-prolyl mesochlorin e₆
5-hydroxy lysyl mesochlorin e₆
y-aminobutanoyl mesochlorin e₆
3-methyl histidyl mesochlorin e₆

### Porphyrin Derivatives

(D,L)-Serinylmesoporphyrin IX
Glycylmesoporphyrin IX
a-(DL)-Alanylmesoporphyrin IX
β-Alanylmesoporphyrin IX
_{E}-Amino-n-caproylmesoporphyrin IX
(D,L)-Serinylprotoporphyrin IX
Glycylprotoporphyrin IX
a-(D,L)-Alanylprotoporphyrin IX
β-Alanylprotoporphyrin IX
_{E}-Amino-n-caproylprotoporphyrin IX
(D,L)-Serinyldeuteroporphyrin IX
Glycyldeuteroporphyrin IX
a-(D,L)-Alanyldeuteroporphyrin IX
β-Alanyldeuteroporphyrin IX
_{E}-Amino-n-caproyldeuteroporphyrin IX
tetra- (D,L)-Serinylcoproporphyrin III
tetra- Glycylcoproporphyrin III
tetra- a-(D,L)-Alanylcoproporphyrin III
tetra- β-Alanylcoproporphyrin III
tetra- _{E}-Amino-n-caproylcoproporphyrin III
(D,L)-Serinylhematoporphyrin IX
Glycylhematoporphyrin IX
a-(D,L)-Alanylhematoporphyrin IX
β-Alanylhematoporphyrin IX
_{E}-Amino-n-caproylhematoporphyrin IX

### Bacteriochlorin Derivatives

(D,L)-Serinylbacteriochlorin e₄
Glycylbacteriochlorin e₄
a-(DL)-Alanylbacteriochlorin e₄
β-Alanylbacteriochlorin e₄
_{E}-Amino-n-caproylbacteriochlorin e₄
(D,L)-Serinylbacterioisochlorin e₄
Glycylbacterioisochlorin e₄
a-(DL)-Alanylbacterioisochlorin e₄
β-Alanylbacterioisochlorin e₄
_{E}-Amino-n-caproylbacterioisochlorin e₄
(D,L)-Serinylbacteriochlorin e₆
Glycylbacteriochlorin e₆
a-(DL)-Alanylbacteriochlorin e₆
β-Alanylbacteriochlorin e₆
ε-Amino-n-caproylbacteriochlorin e₆

Other amino acid derivatives of the tetrapyrroles can also be prepared. The following amino acids can also be used to prepare the mono- di-, tri-, or where appropriate, the tetra-amino acid derivatives of the chlorins, porphyrins, or bacteriochlorins, employing the procedures of one of the aforementioned methods: piperidine-2-carboxylic acid, piperidine-6-carboxylic acid, pyrrole-2-carboxylic acid, pyrrole-5-carboxylic acid, piperidine-6-propionic acid, and pyrrole-5-acetic acid

Mixed amino acid derivatives of the tetrapyrroles can also be prepared. The various chlorin derivatives, porphyrin derivatives and bacteriochlorin derivatives can include any two or three of the following amino acids: glycine, serine, threonine, cysteine, tyrosine, asparagine, glutamine, lysine, arginine, histidine, a-alanine, β-alanine, valine, leucine, isoleucine, proline, a-phenylalanine, β-phenylalanine, tryptophan, methionine, ε-amino-n-caproic acid, piperidine-2-carboxylic acid, pyrrole-5-carboxylic acid, piperidine-6-propionic acid, pyrrole-5-acetic acid.

Physical characteristics of the compounds (relative polarity) is measured by a standard chromatographic system. The chromatographic data (Rf values) were measured on Baker silica gel-C18 thin layer chromatographic plates, the particle size of which is 20 µM, and the coating thickness of which is 200 µM. The solvent system for these chromatographic runs consisted of 75% methanol, and 25% 0.01 M potassium phosphate buffer, pH 6.85. The compounds were spotted and dried on the plate as the sodium salts, at approximately neutral pH and minimum salt concentrations. The Rf values for the various derivatives are tabulated in TABLE 1. Spectroscopic data are indicated in TABLE 2.

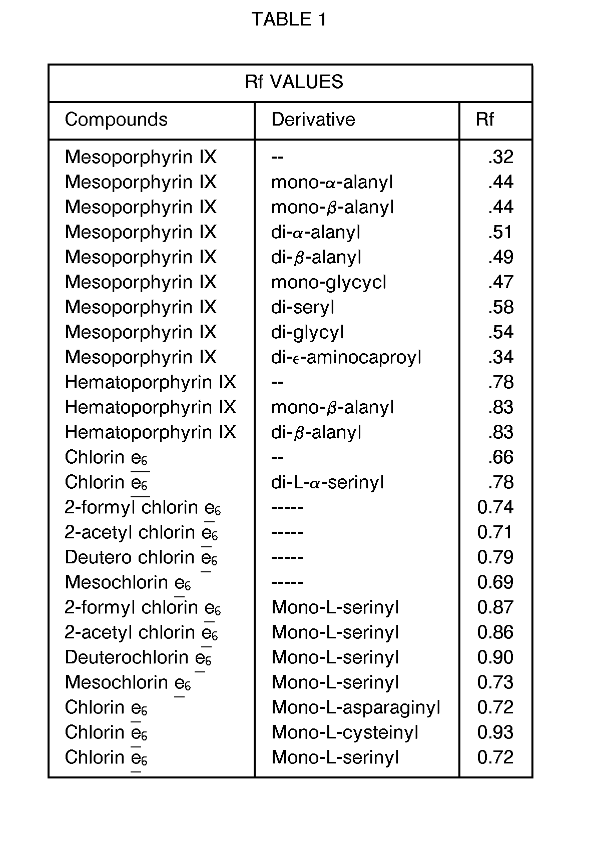

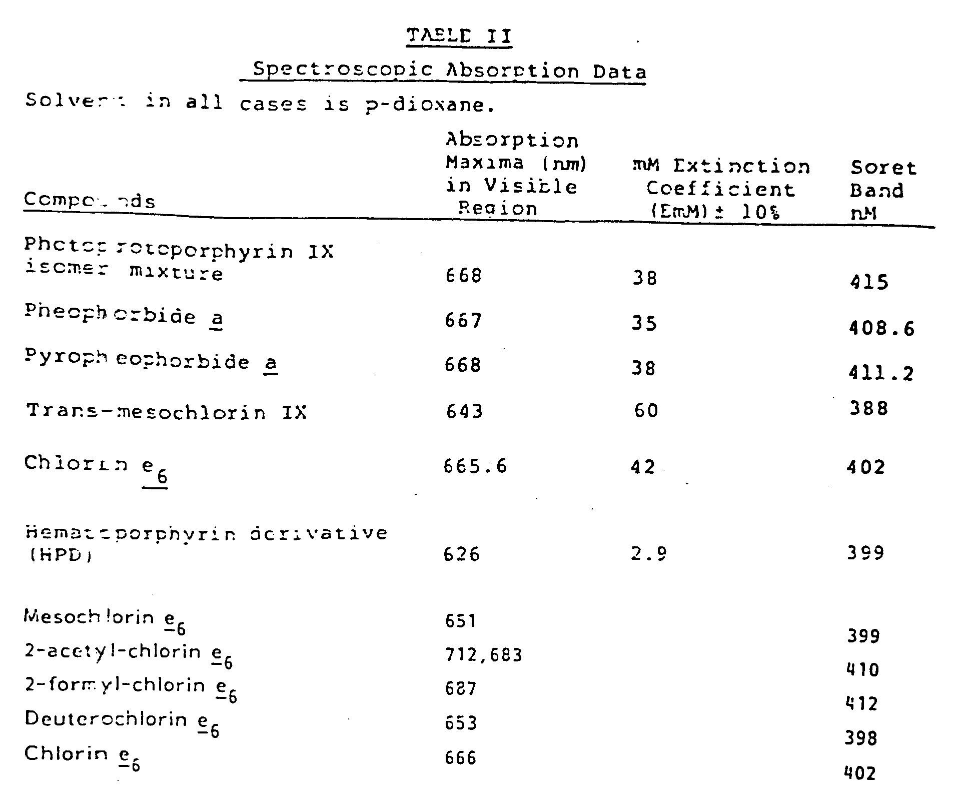

Absorption data for the amino acid conjugates is identical to the parent chlorins.

The following protocol describes the procedure for the utilization of these new compounds of the present invention in the treatment of rat tumors.

### EXAMPLE XXII

The photodynamic therapy experiments have been carried out on Buffalo rats, using the transplantable tumor, Morris Hepatoma 7777. The tumors were transplanted subcutaneously on the outside of the thigh. During treatment, the tumors ranges in size between 1 and 2.5 cm in diameter.

The general treatment regime is as follows. The rats are injected with a solution of the chlorin prepared as follows: 20 mg of the sodium salt of the chlorin was dissolved in 1 ml of 0.9% NaCI. The chlorin solution was then injected intravenously through the external jugular while the rat was anesthetized with ether. The volume of solution injected was calculated based upon the weight of the animal and the dosage, on a weight to weight basis, for the particular experiment. A specified time interval was then allowed to elapse before light treatment was instigated.

Light treatment of the rats was without anesthesia. The rats were restrained, the hair removed in the treatment area and treated with laser light from a Cooper Aurora argon pumped, tunable dye laser.

The laser was equipped with a fiber optic light delivery system coupled to a microlens system developed by Dr. Daniel Doiron, D.R.D. Consulting, Santa Barbara, California.

The lens disperses the laser beam, providing a circular distribution of light with homogenous light intensity throughout the area of the incident light beam. The wavelength of light was adjusted using a Hartridge reversion spectroscope. The light intensity was determined using a Yellow Springs Instrument, Model 65A, radiometer.

The micro lens was positioned at such a distance from the skin of the animal so as to provide an illumination diameter of 1.5cm, and the light flux was varied by control of the laser output.

Subsequent to illumination, the animal was returned to its cage and, 24 hours later, it was treated intravenously in the external jugular vein with 14 mg of Evans Blue dye, dissolved in 250 µl of 0.9% NaCI. Two hours after injection, the rat was sacrificed and the tumor cross-sectioned. The extent of tumor necrosis was assessed by the lack of dye uptake (¹), and the depth of the necrotic cross section of the tumor was recorded in millimeters.

Table III summarizes the effects of these drugs on tumors and includes a range of wavelengths, dosages, intensities, and time intervals for treatment. This has been necessary, in order to attempt to establish the optimal conditions for phototherapy utilizing this new drug. The conditions described result in measurable and significant damage to the tumors.

In all cases except where noted, tissue damage occurred selectively to the tumor tissue as assayed by the Evans Blue method, even though, in nearly all cases, normal skin overlayed the tumor and the treatment area overlapped significant areas of normal muscle tissue.

The photodynamic therapy date is presented in tabular form. Column No. 2 is the total light dose administered in terms of Joules per square centimeter. Column No. 3 is the dose of chlorin administered in terms of mg of drug per kilogram of rat body weight. Column No. 4 is the time lapse between administration of drug and treatment with laser light. Column No. 5 is the wavelength of treatment light in nanometers. Column No. 6 is the intensity of the treatment light in milliwatts per square centimeter. In Column No. 7, x is the mean depth of necrosis in millimeters of the tumor tissue, i.e., the distance from the necrotic top of the tumor next to the skin to the necrotic edge of the tumor most distant from the skin.

S.D. is the standard deviation of x.

(N) is the number of tumors or legs involved in the experiment.

Column No. 8 is the range of depth of necrosis in millimeters within the group.
(1) M.C. Berenbaum, Br. J. Cancer 45 : 571 (1982)

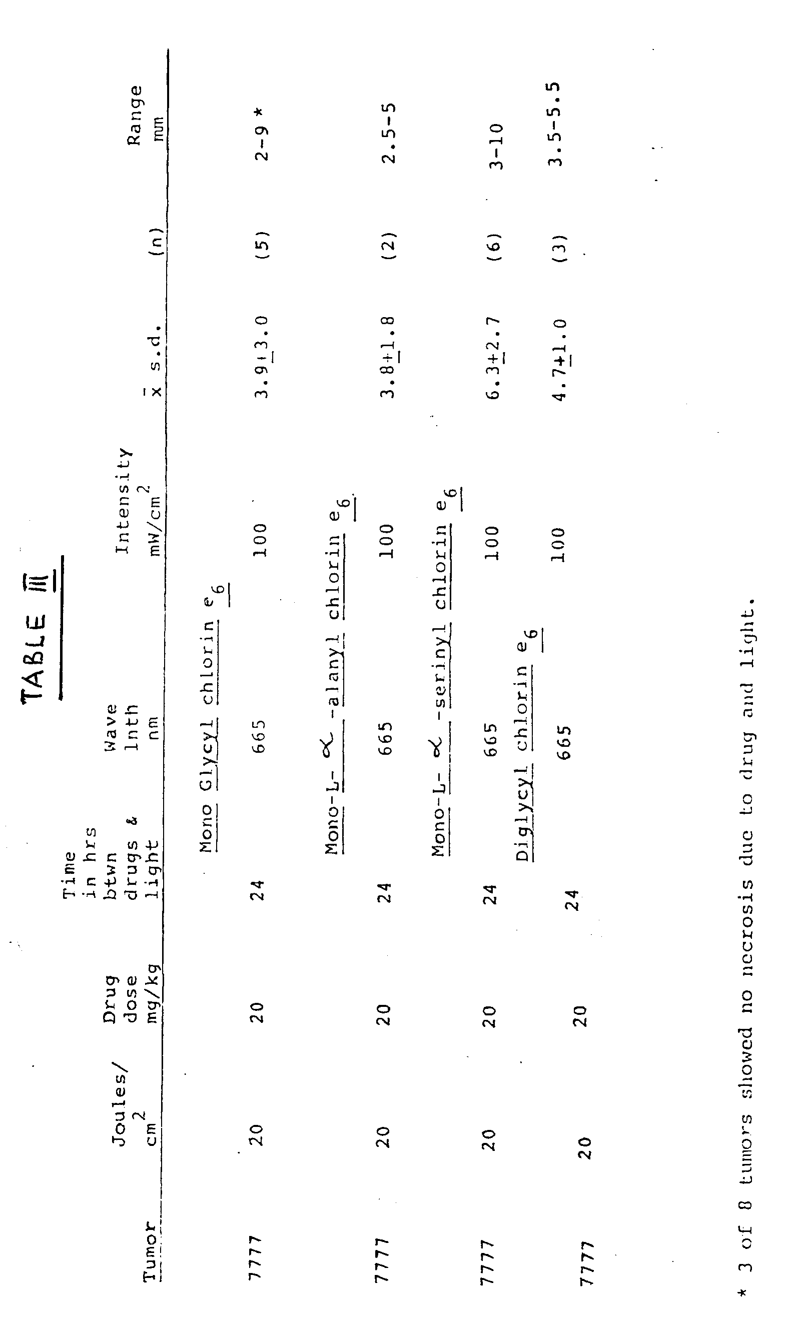

### EXAMPLE XXIII

The treatment and evaluation procedure is as follows:

DBA/2 Ha Ros-d + Ha mice with SmT-F transplanted tumors either in the exterior part of the hind leg or the side of the mouse were injected intravenously via the external jugular or the intraperitoneally with the photosensitizing drug. At the specified time after injection, the area over the tumor was shaved and the light treatment begun.

Light from a Copper Aurora argon pumped tunable dye laser was administered via a micro lens system (developed by Dr. Daniel Doiron, D.R.D. Consulting, Santa Barbara, California) coupled through a quartz fiber to the laser, the optical properties of the lens are such that the light exits the lens in a circular pattern with homogenous intensity throughout the lighted area. The diameter of the lighted area is a function of the distance from the lens.

The light intensity was measured with a Yellow Springs Instrument Model 65 A Radiometer at the point of treatment. A 1.5 cm diameter circle of the animal's skin, centered as closely as possible over the tumor, was irradiated in all the experiments. The intensity, wavelength, and dosage of light is included in the data for individual groups of animals. Wavelengths are adjusted, using a Hartridge reversion spectroscope to within 1 nm of the stated value.

Twenty four hours after light treatment, each mouse received 5 mg of Evans Blue Dye intravenously (¹). After an additional two hours, the mice were sacrificed and the tumors were sectioned vertically through the center of the light treated area. Unaffected tumor was stained blue as was unaffected normal tissue. Necrotic or affected areas were white or red in appearance. Measurements on both the whole tumors and affected areas of the tumors were made vertically and horizontally with calipers to the nearest one half millimeter. The results of representative compounds are depicted in the following tables:
(1) M.C. Berenbaum. Br. J. Cancer. 45:571 (1982).

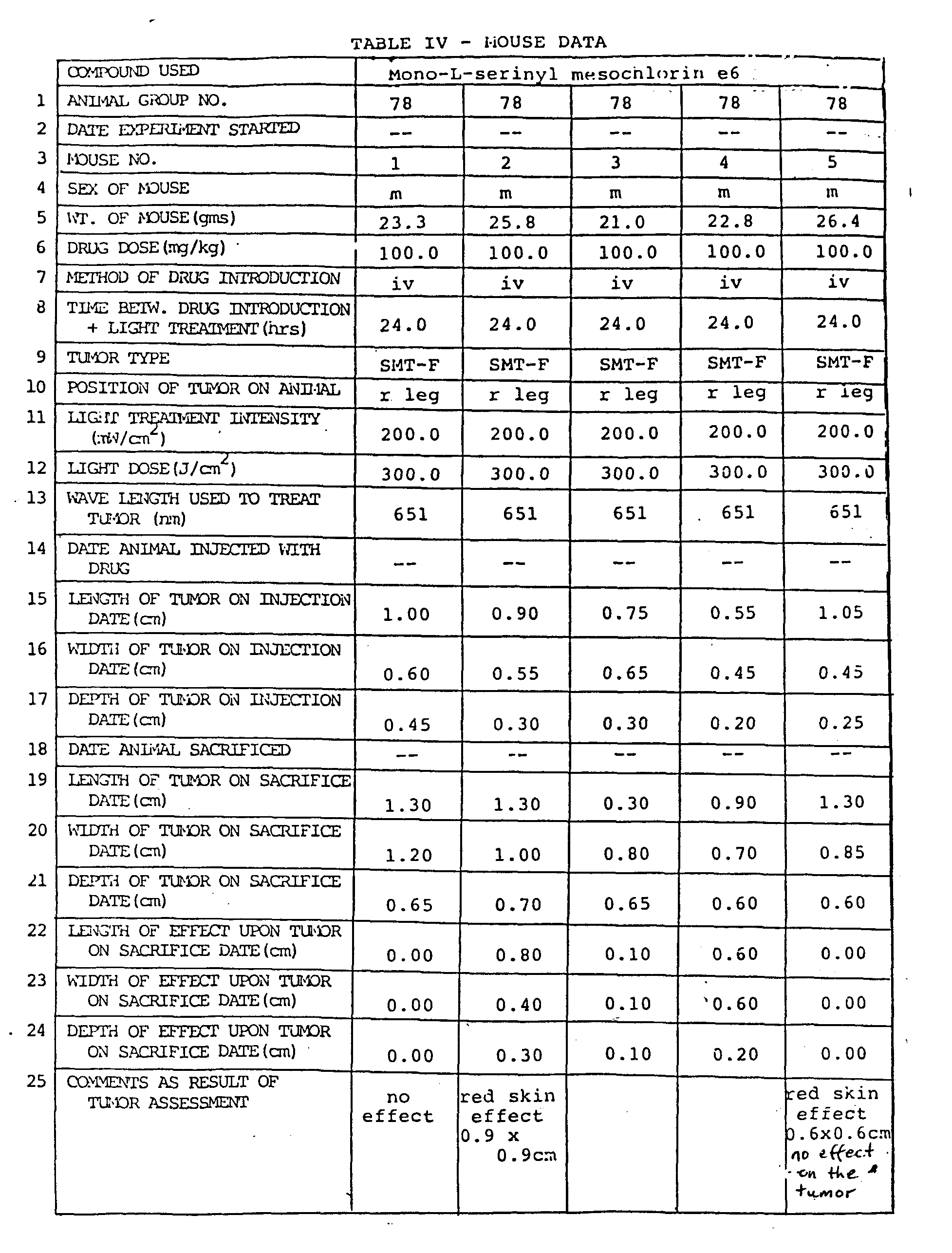

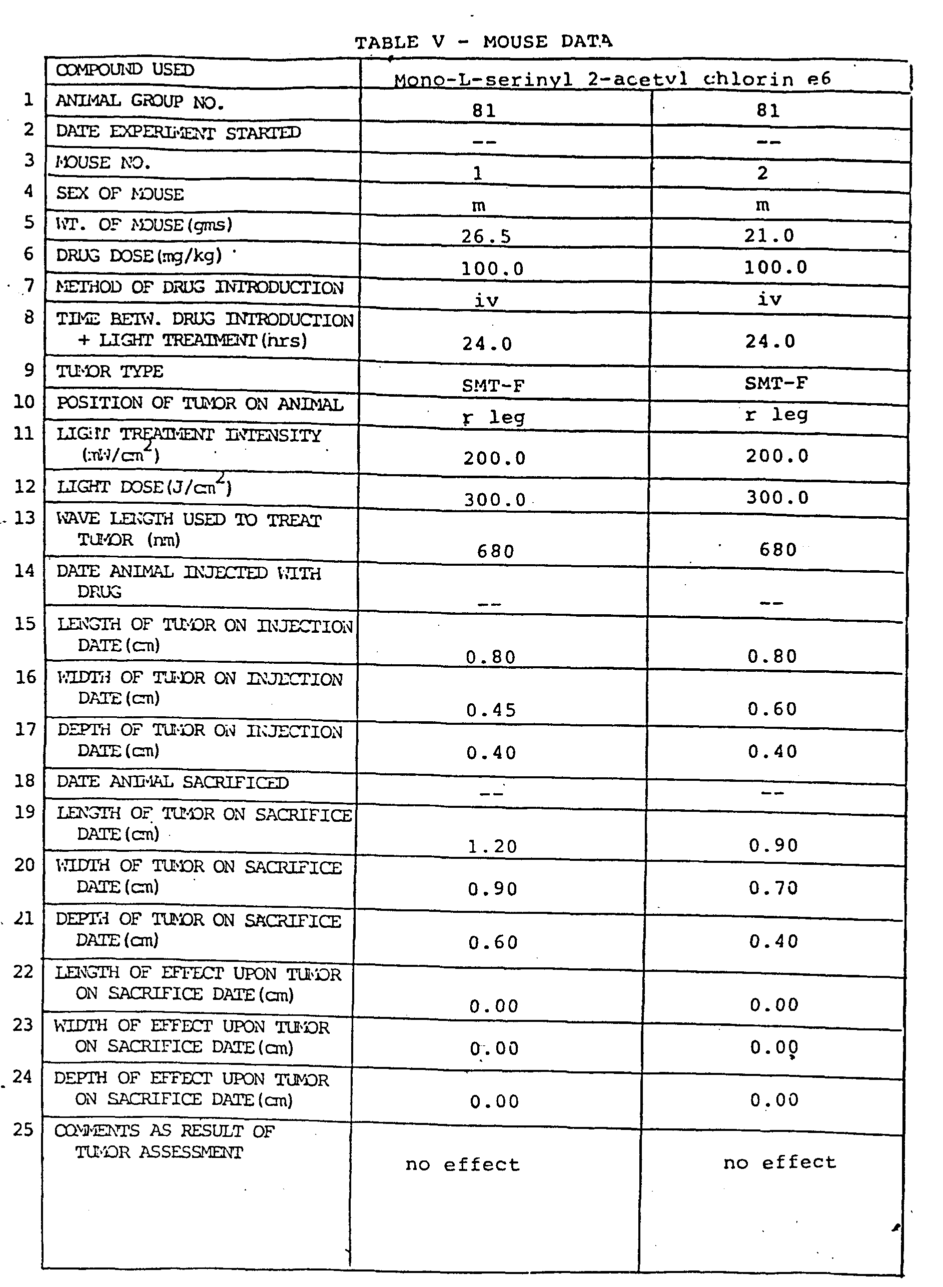

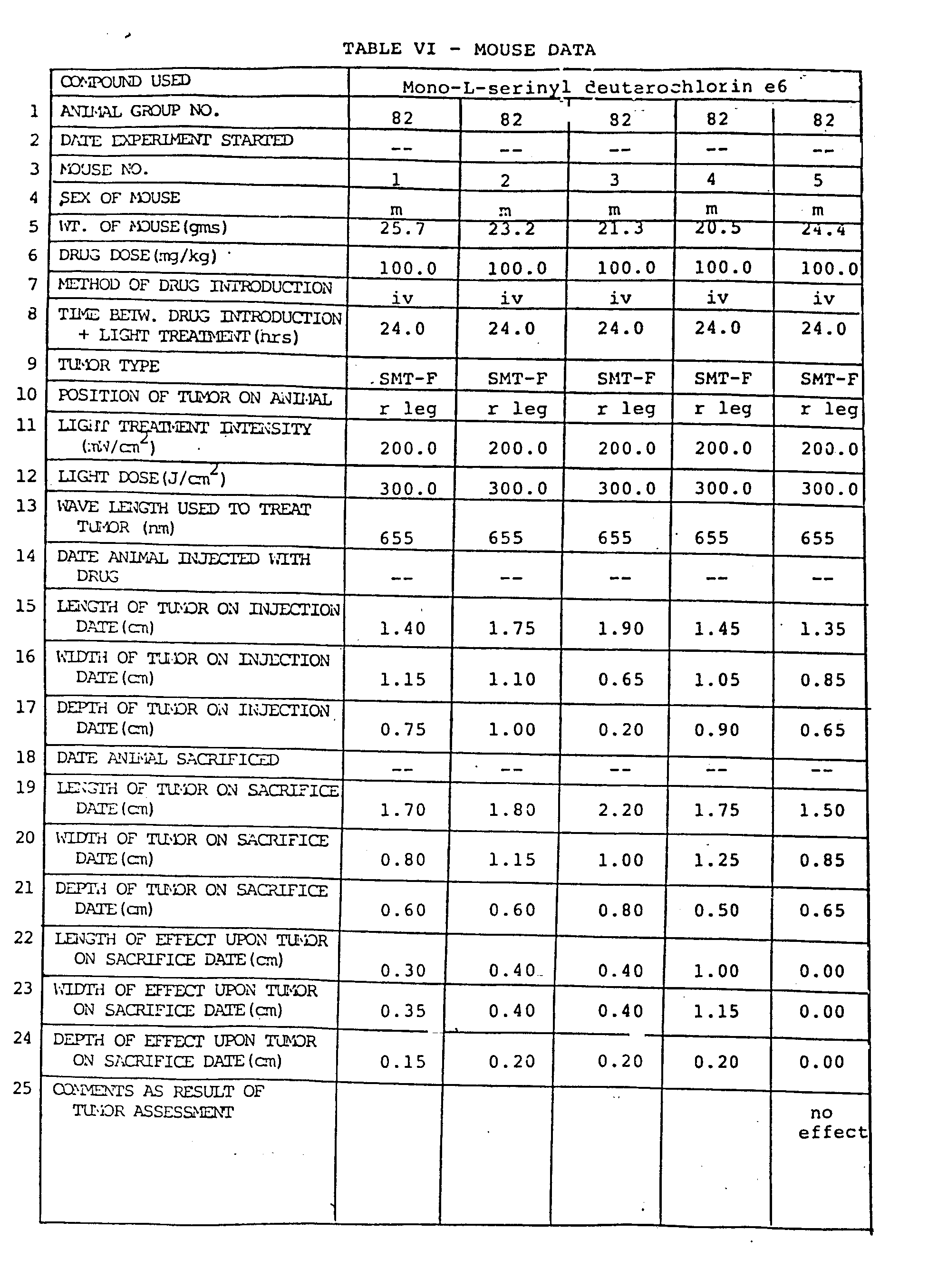

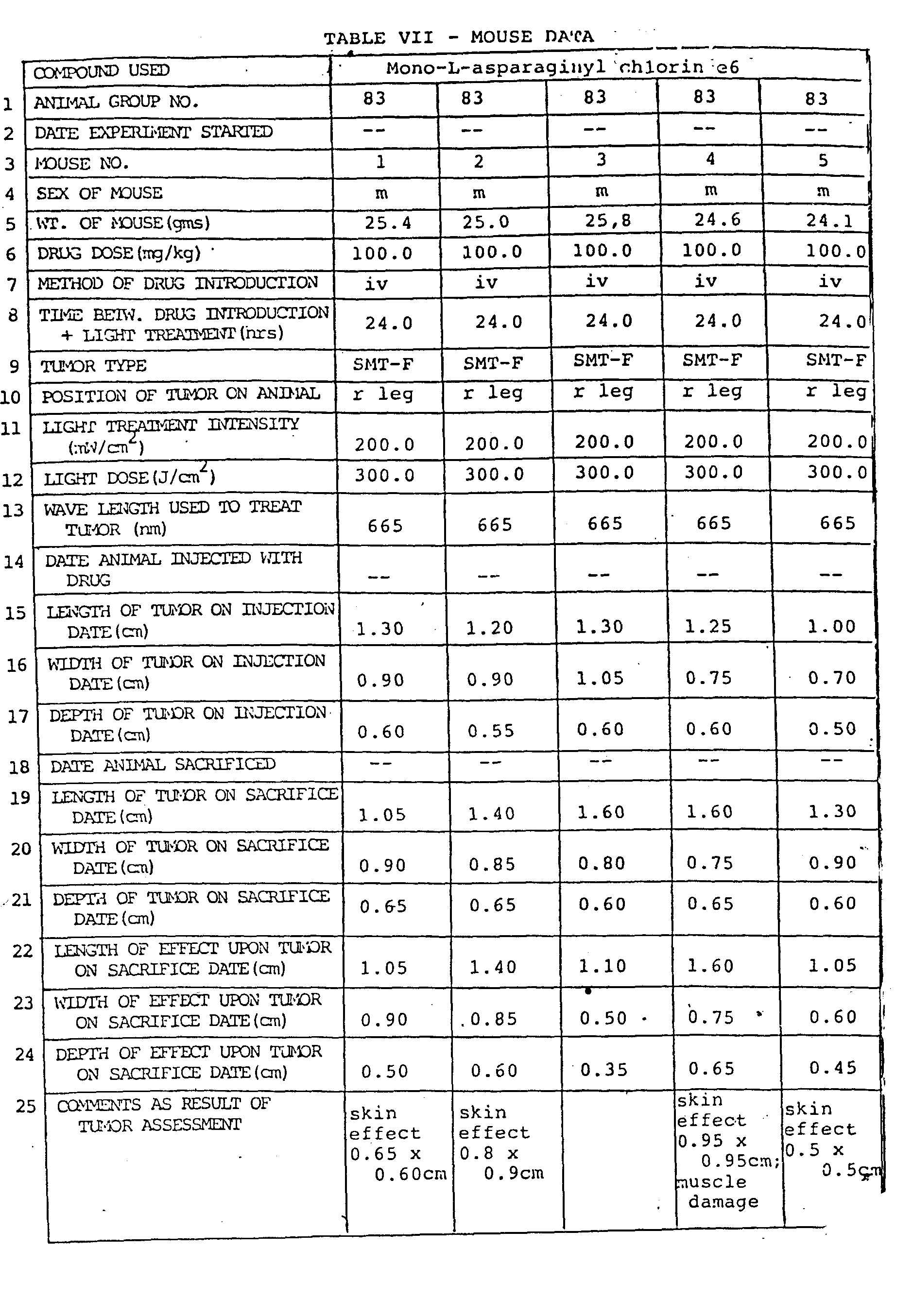

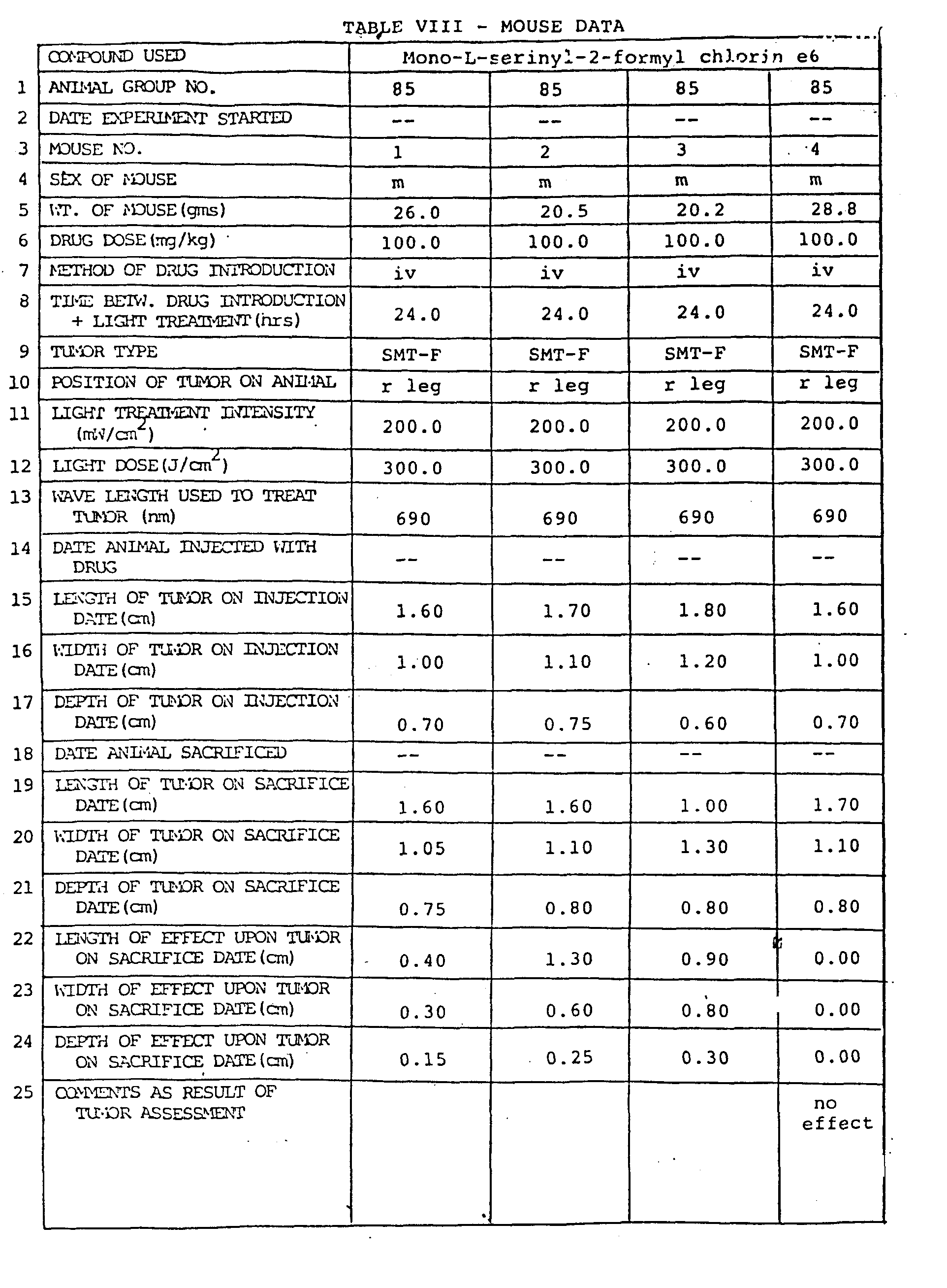

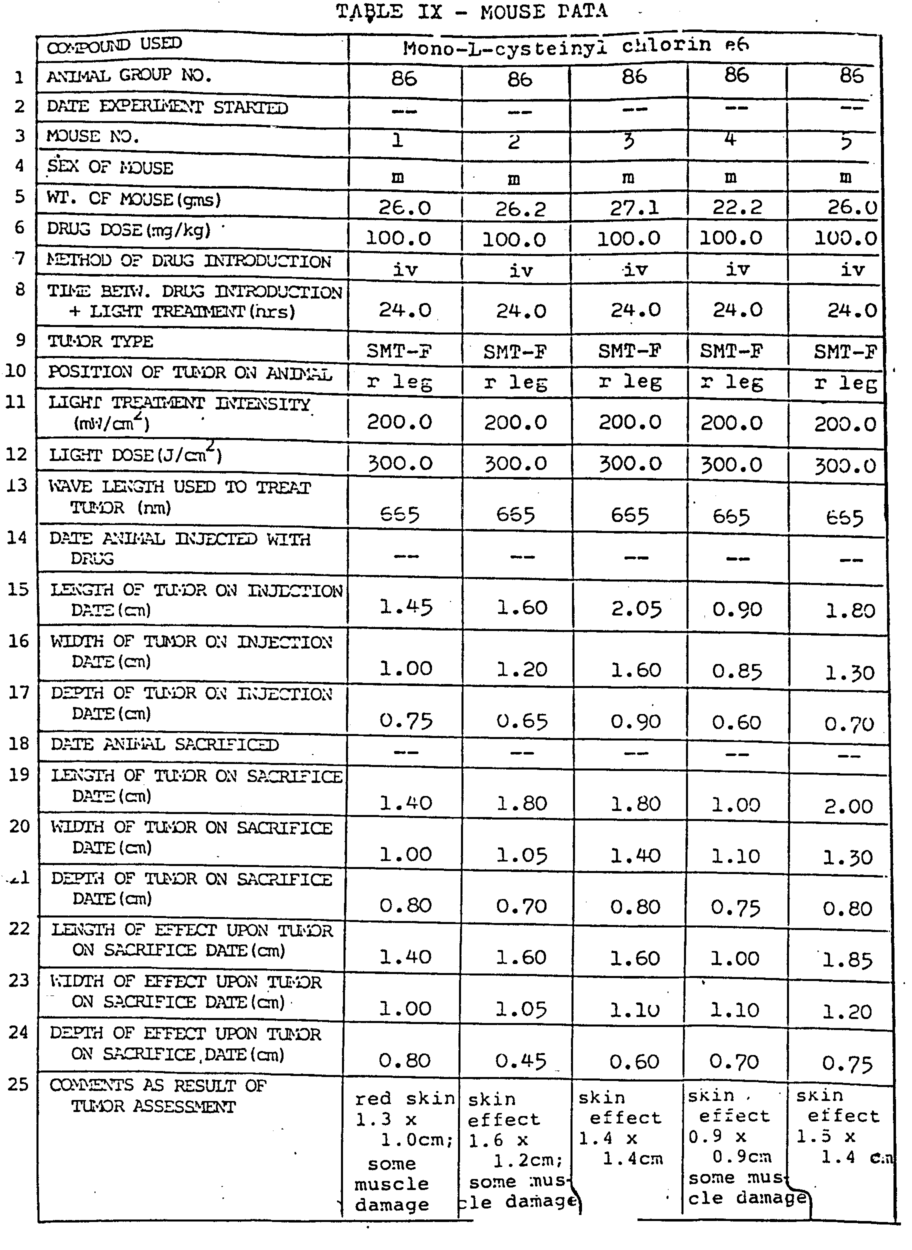

The results of Table IV - IX are summarized in Table X.

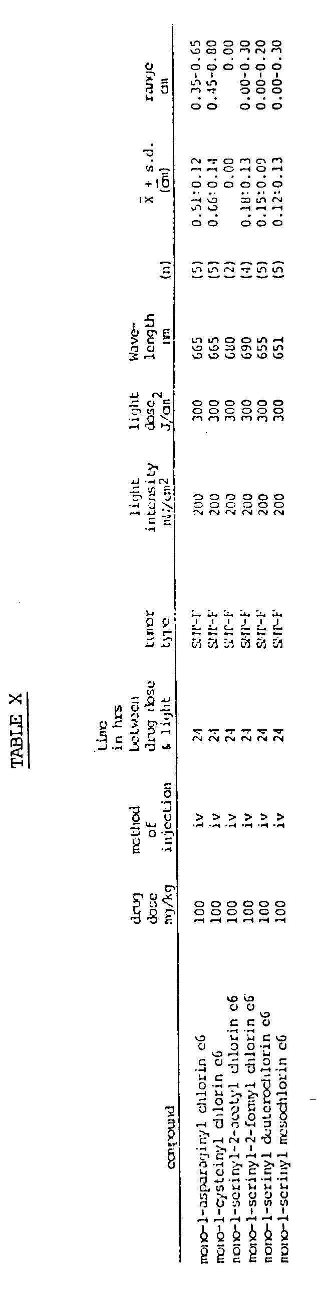

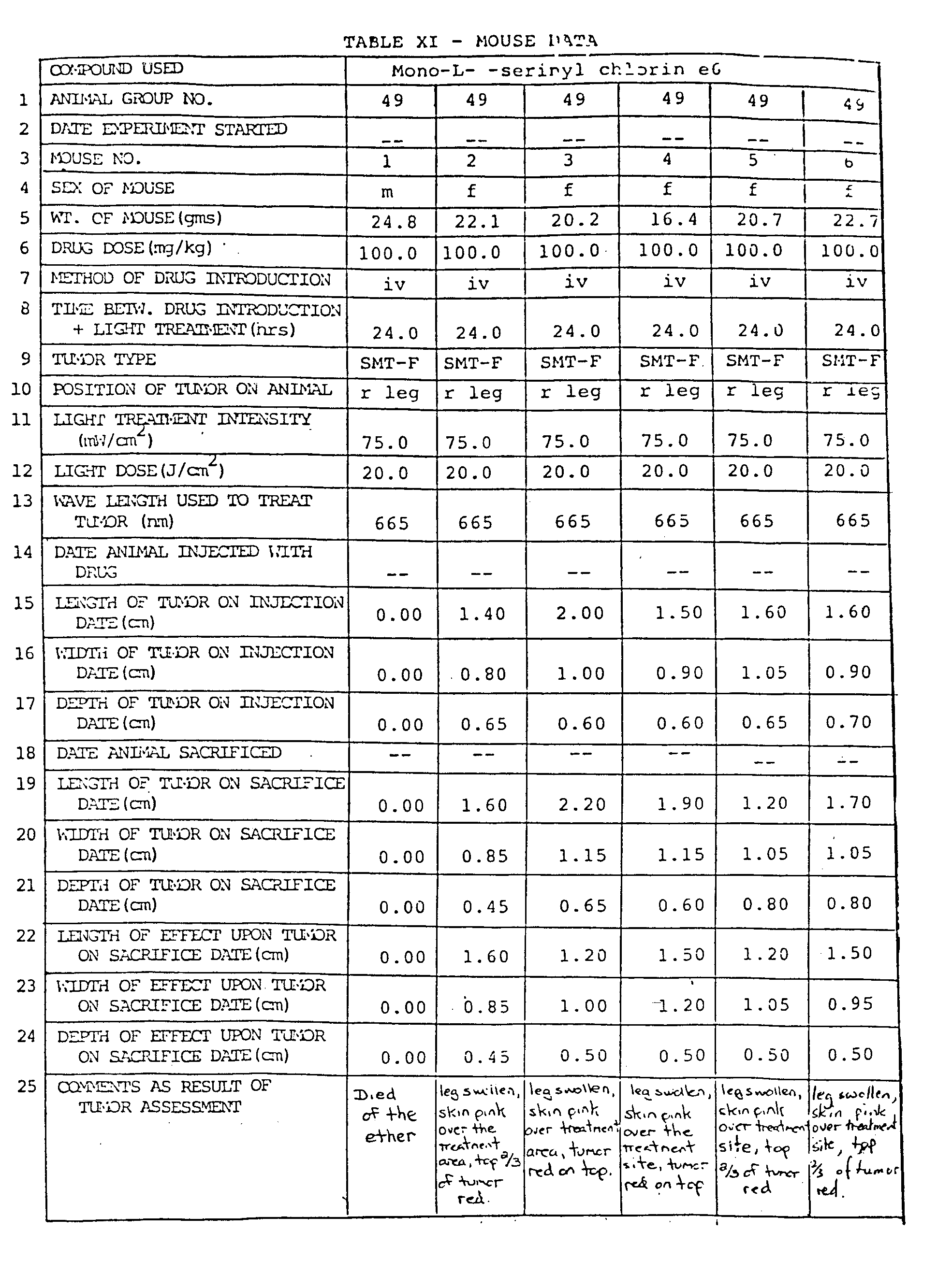

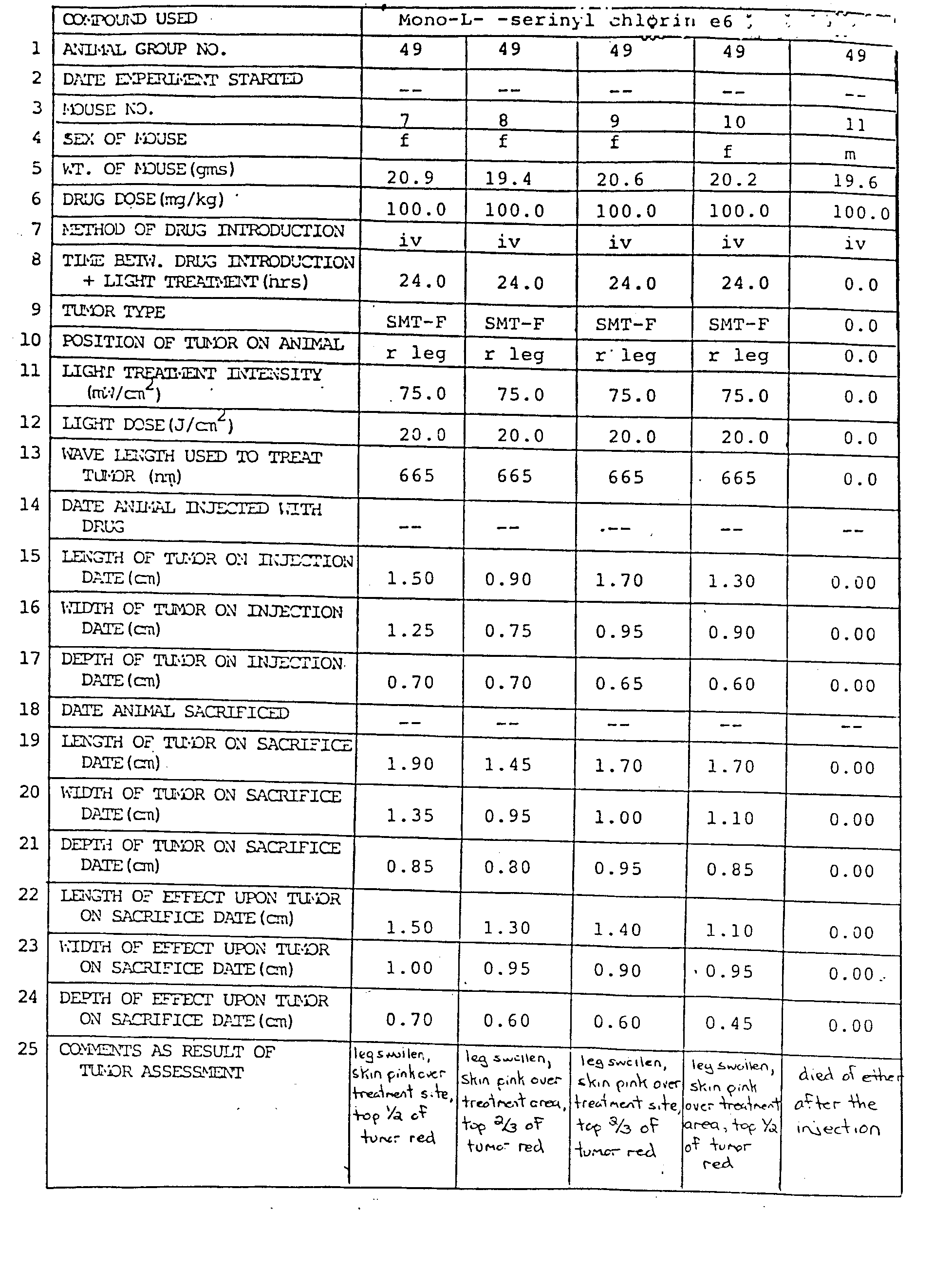

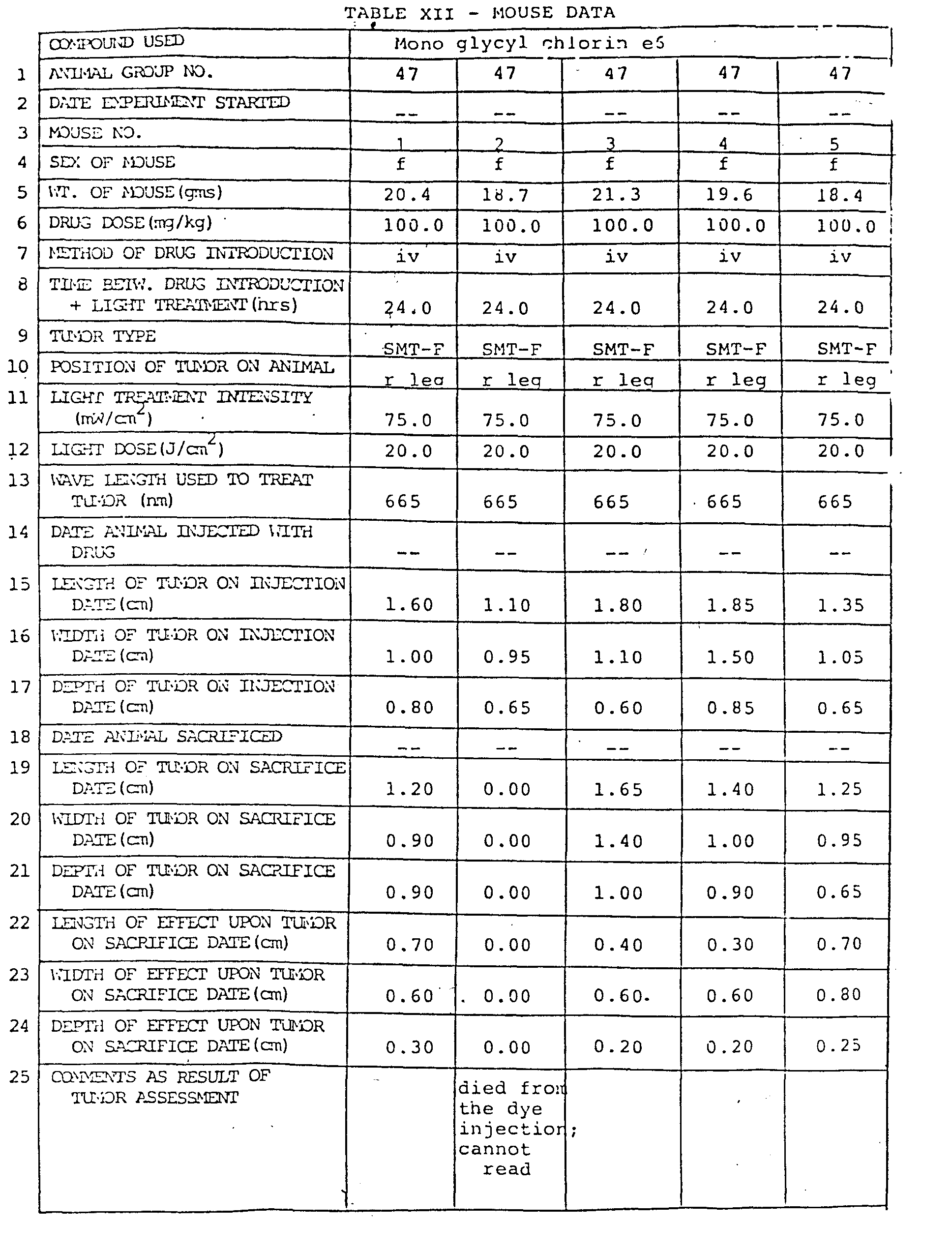

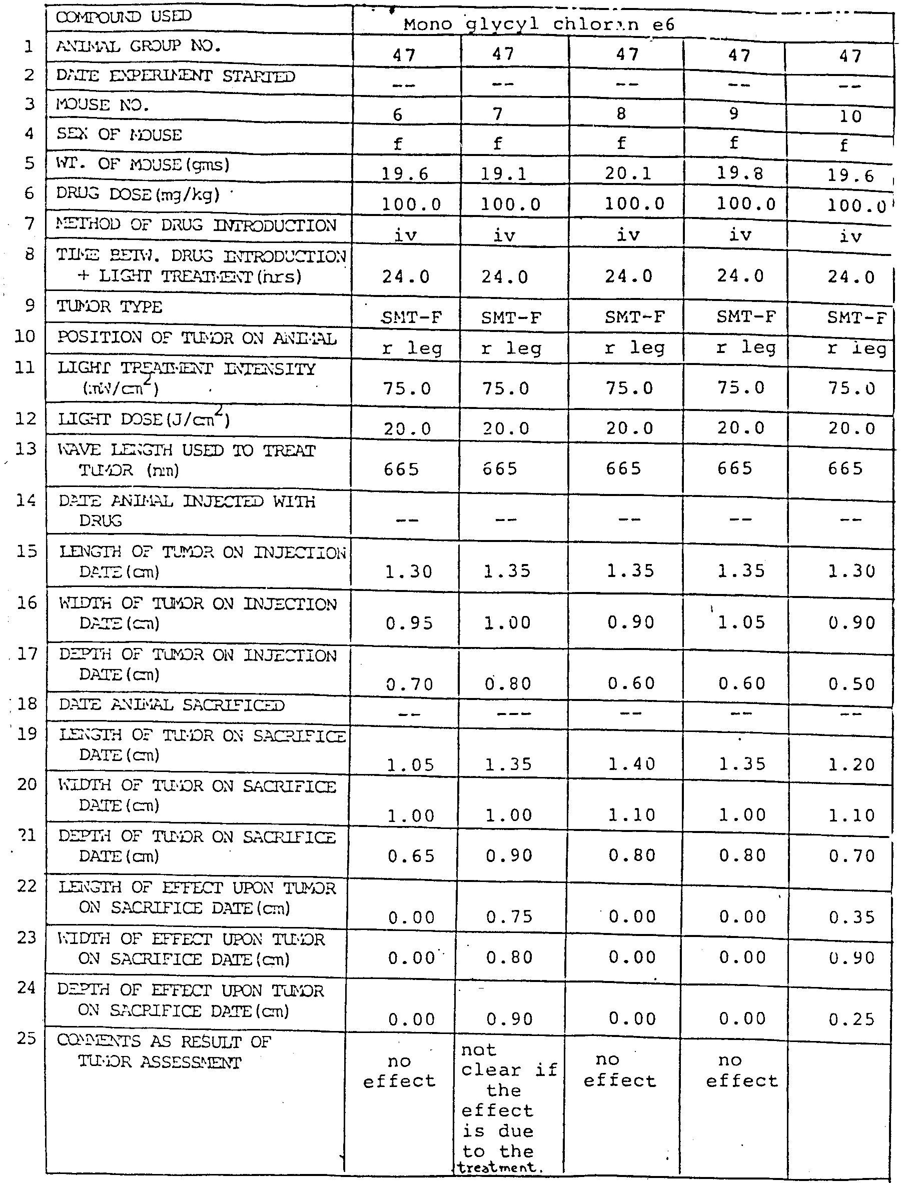

The results Of Table XI - XII are summarized in Table XIII.

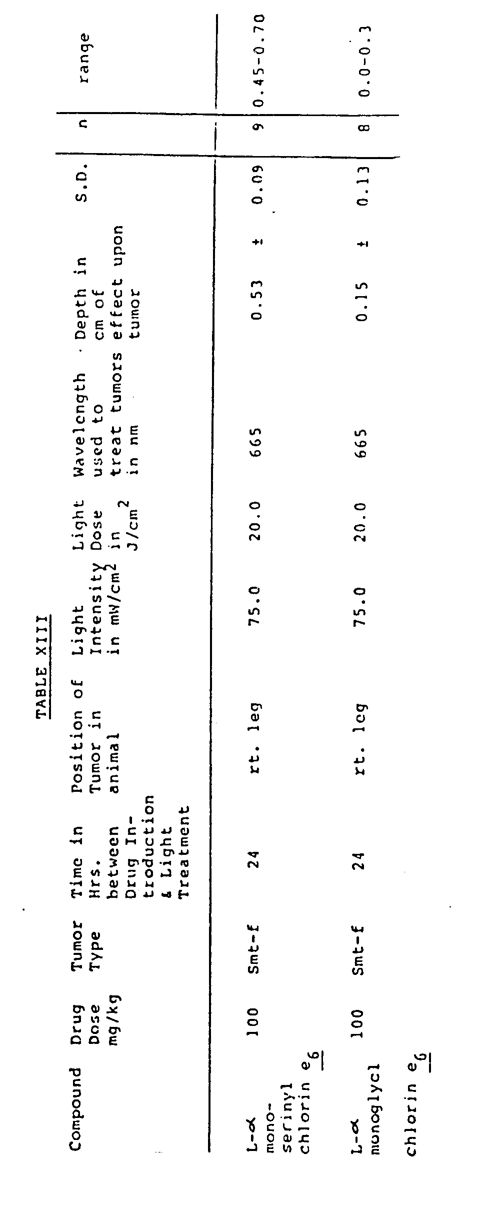

The preparation of pharmacological dosages for the administration of the active ingredient, that is the amino acid porphyrin adducts, which were prepared in Examples 1-21 hereinabove, is as follows:

### EXAMPLE XXIV

A tablet base was prepared by blending the following ingredient in the proportion by weight indicated:

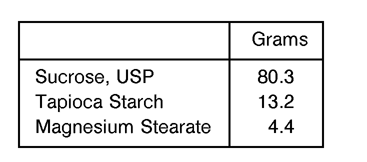

Into this base, there was blended sufficient amino acid porphyrin adducts to provide tablets each containing 100 mg. of active ingredient.

### EXAMPLE XXV

A blend was prepared containing the following ingredients:

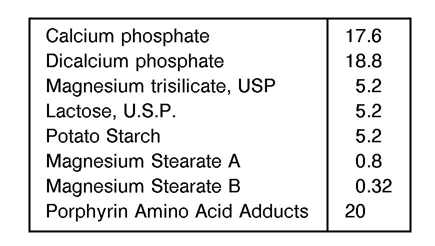

This blend was divided and formed into capsules each containing 25 mg of active ingredient.

### EXAMPLE XXVI

To a commercially available raspberry flavored sugar syrup is added the equivalent of 40 mg of the amino acid porphyrin adduct per milliliter and the mixture is homogenized in a mechanical device for this purpose. This mixture is especially suitable for oral administration containing 200 mg of the active ingredient.

### EXAMPLE XXVII

A sterile solution of the following composition is prepared: 200 mg of the sodium salt of the amino acid porphyrin adduct is dissolved in a 0.9% NaCl solution so that the final concentration is 20 mg/ml.

This solution is suitable for I.V. and I.M. administration.

### EXAMPLE XXVIII

The sodium salt of the amino acid porphyrin adduct is dissolved in 0.9% NaCl solution so that the final concentration is 5 mg/ml. This is placed in an aerosal dispenser with a hydrocarbon propellant. This preparation is suitable for topical application.

### EXAMPLE XXIX

### PREPARATION OF A METAL SALT

The sodium salt of the porphyrin amino acid adduct is prepared by dissolving said adduct in water containing an equimolar amount of sodium hydroxide and freeze drying the resulting mixture.

In this fashion, other metal salts are prepared including potassium, calcium, and lithium salts.

### PREPARATION OF AN ACID SALT

The amino acid porphyrin adduct described in the preceding examples are converted to acid salts, e.g., hydrochloride, by dissolving in an aqueous solution containing an equivalent amount of acid, e.g., hydrochloric acid, and the solution is evaporated to dryness to obtain the solid salt. Alternately, alcoholic solutions of hydrogen chloride gas, dissolved in ethanol can be used in lieu of the aqueous acid solution and the acid salt is obtained by evaporation of the solvent or crystallization from the alcohol, e.g., by addition of a non-solvent.

## Claims (Claims for the following Contracting State(s) : BE, CH, DE, FR, GB, IT, LI, LU, NL, SE)

1. A therapeutic composition comprising a fluorescent mono, di or polyamide of an aminomonocarboxylic acid and a tetrapyrrole compound of the formula: 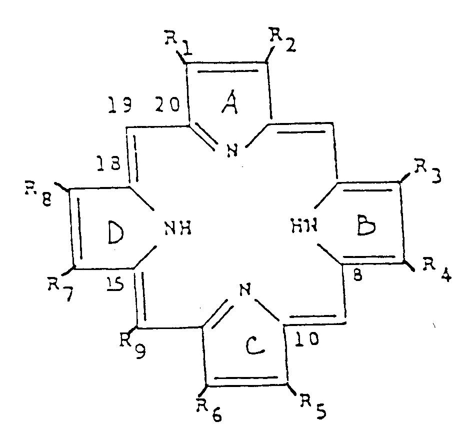 or salt thereof
or the corresponding di- or tetrahydrotetrapyrroles and a pharmaceutically acceptable carrier therefor; wherein
R₁ is methyl; 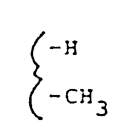 or 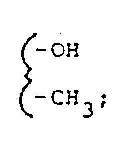
R₂ is H, vinyl, ethyl, 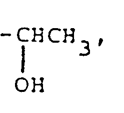 acetyl, 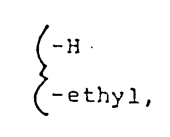 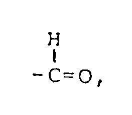 CH₂CH₂CO₂H, or =CHCHO;
R₃ is methyl 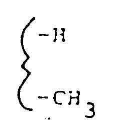 or 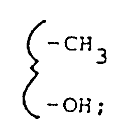
R₄ is H, vinyl, ethyl, 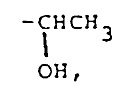 CH₂CH₂CO₂H, =CHCHO; or 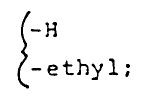
R₅ is methyl;
R₆ is H, CH₂CH₂CO₂H, CH₂CH₂CO₂R or CO₂H;
R₇ is CH₂CH₂C0₂H, CH₂CH₂C0₂R, or 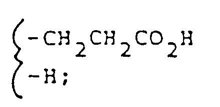
R₈ is methyl or
Rg is H, COOH, CH_{2COO}H or methyl;
provided that when Rᵢ, R₂, R₃, R₄, R₇ and R₈ represent two substituents or are divalent and attached to the same carbon, the respective pyrrole ring to which they are attached is a dihydropyrrole;
R is lower alkyl or benzyl;
with the proviso that at least one of R₁ -Rg includes a free carboxyl group, and that amide bonds (1 to 4) are formed between the amino group of the aminomonocarboxylic acid and one of the carboxy groups of the tetrapyrrole; with the proviso that the tetrapyrrole residue is not pheophorbide a.

2. The composition according to claim 1 wherein the amino acid is an alpha amino acid.

3. Composition according to claim 1 or 2 wherein the tetrapyrrole is a porphyrin, chlorin or bacteriochlorin.

4. Composition according to any of claims 1 to 3, wherein the amide-containing substituents are asymmetrically arranged on the tetrapyrrole molecule.

5. Composition according to claim 1 wherein the amide is:
diserinyl mesophorphyrin IX,
diglycyl mesoporphyrin IX,
di-a-(DL)-alanyl mesoporphyrin IX,
di-β-alanyl mesoporphyrin IX,
di-_{E}-amino-n-caproyl mesoporphyrin IX,
diglycyl trans-mesochlorin IX,
diglycyl trans-mesochlorin e₆,
diglycyl mesochlorin e₄,
diglycyl hematoporphyrin IX,
diglycyl chlorin e₆,
diglycyl protoporphyrin IX,
diglycyl deuteroporphyrin,
di-a-(DL)-alanyl trans-mesochlorin IX,
di-a-(DL)-alanyl mesochlorin e₆,
di-a-(DL)-alanyl mesochlorin e₄,
di-a-(DL)-alanyl hematoporphyrin IX,
di-a-(DL)-alanyl chlorin e₆,
di-a-(DL)-alanyl protoporphyrin IX,
di-a-(DL)-alanyl deuteroporphyrin,
di-β-(DL)-alanyl trans-mesochlorin IX,
di-β-(DL)-alanyl mesochlorin e₆,
di-β-(DL)-alanyl mesochlorin e₄,
di-β-(DL)-alanyl hematoporphyrin IX,
di-β-(DL)-alanyl chlorin e₆,
di-β-(DL)-alanyl protoporphyrin IX,
di-β-(DL)-alanyl deuteroporphyrin,
di-L-a-serinyl chlorin e₆,
di-L-a-serinyl trans-mesochlorin e₆,
di-L-a-serinyl trans-mesochlorin IX,
di-L-a-serinyl trans-mesochlorin e₄,
di-L-a-serinyl hematoporphyrin IX,
di-L-a-serinyl protoporphyrin IX,
di-L-a-serinyl deuteroporphyrin,
di-_{E}-amino-n-caproyl-hematoporphyrin IX,
di-_{E}-amino-n-caproyl-chlorin e₆,
di-_{E}-amino-n-caproyl-protoporphyrin IX,
di-_{E}-amino-n-caproyl-deuteroporphyrin,
mono-L-serinyl mesochlorin e₆,
mono-L-serinyl Deuterochlorin e₆,
mono-L-serinyl-2-formyl chlorin e₆,
mono-L-serinyl-2-acetyl chlorin e₆,
mono-L-cysteinyl chlorin e₆,
mono-L-asparaginyl chlorin e₆
mono serinyl chlorin e₆,
mono-(DL)glycyl chlorin e₆,
alanyl chlorin e₆,
mono-L-valyl chlorin e₆,
mono-L-leucyl chlorin e₆,
mono-L-isoleucyl chlorin e₆,
mono-L-prolyl chlorin e₆,
mono-L-methionyl chlorin e₆,
mono-L-threoninyl chlorin e₆,
tyrosyl chlorin e₆,
glutaminyl chlorin e₆,
lysyl chlorin e₆,
arginyl chlorin e₆,
histidyl chlorin e₆,
β-alanyl chlorin e₆,
mono-ε-amino-n-caproyl chlorin e₆,
monoglycyl mesoporphyrin IX,
mono alanyl mesoporphyrin IX,
mono-β-alanyl mesoporphyrin IX,
mono-E-amino-n-caproyl mesoporphyrin IX,
mono-β-alanyl-hematoporphyrin IX,
threoninyl-2-formylchlorin e₆,
mono-L-threoninyl deuterochlorin e₆, or
mono-L-threoninyl mesochlorin e₆,

6. Use of a fluorescent mono, di or polyamide of an aminomonocarboxylic acid and a tetrapyrrole containing one or two carboxy groups of the structure as claimed in any of claims 1 to 5, or a salt thereof, and a pharmaceutically acceptable carrier therefor, for the preparation of a therapeutic composition for photodiagnosis and/or phototherapy of tumors.

## Claims (Claims for the following Contracting State(s) : AT)

1. Use of a fluorescent mono, di or polyamide of an aminomonocarboxylic acid and a tetrapyrrole compound of the formula: 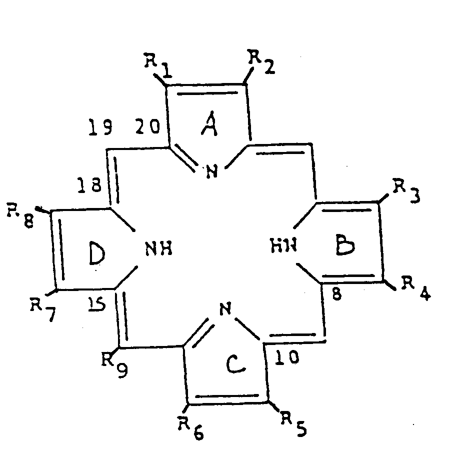 or salt thereof
or the corresponding di- or tetrahydrotetrapyrroles, and a pharmaceutically acceptable carrier therefor; wherein
R₁ is methyl; 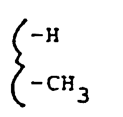 or 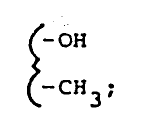
R₂ is H, vinyl, ethyl, 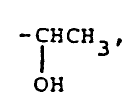 acetyl, 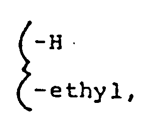 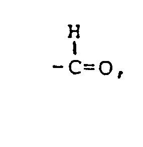 CH₂CH₂CO₂H, or = CHCHO;
R₃ is methyl 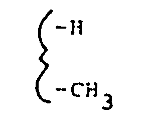 or 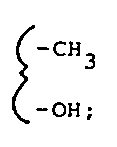
R₄ is H, vinyl, ethyl, 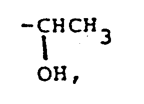 CH₂CH₂CO₂H, = CHCHO; or 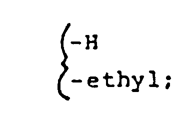
R₅ is methyl;
R₆ is H, CH₂CH₂CO₂H, CH₂CH₂CO₂R or CO₂H;
R₇ is CH₂CH₂C0₂H, CH₂CH₂C0₂R, or 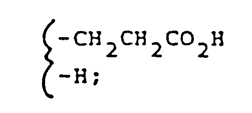
R₈ is methyl or 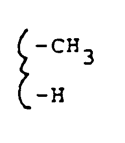
R₉ is H, COOH, CH_{2COO}H or methyl;
provided that when Rᵢ, R₂, R₃, R₄, R₇ and R₈ represent two substituents or are divalent and attached to the same carbon, the respective pyrrole ring to which they are attached is a dihydropyrrole;
R is lower alkyl or benzyl;
with the proviso that at least one of R₁ -R₉ includes a free carboxyl group, and that amide bonds (1 to 4) are formed between the amino group of the aminomonocarboxylic acid and one of the carboxy groups of the tetrapyrrole; with the proviso that the tetrapyrrole residue is not pheophorbide a; for the preparation of a therapeutic composition for photodiagnosis and/or phototherapy of tumors.

2. Use according to claim 1 , characterized in, that the amino acid is an alpha amino acid.

## Patentansprüche (Patentansprüche für folgende(n) Vertragsstaat(en) : BE, CH, DE, FR, GB, IT, LI, LU, NL, SE)

1. Therapeutische Zusammensetzung, enthaltend ein fluoreszierendes Mono-, Di- oder Polyamid einer Aminomonocarbonsäure und einer Tetrapyrrol-Verbindung der Formel 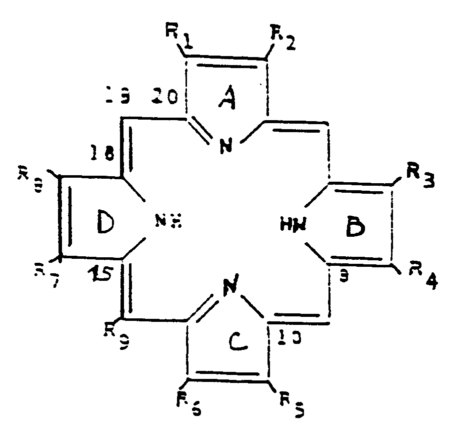 oder eines Salzes davon
oder der entsprechenden Di- oder Tetrahydrotetrapyrrole, und einen pharmazeutisch akzeptablen Träger dafür, wobei
R₁ Methyl, 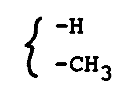 oder 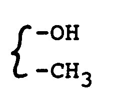 ist;
R₂ Wasserstoff, Vinyl, Ethyl, 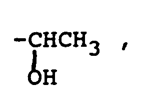 Acetyl, 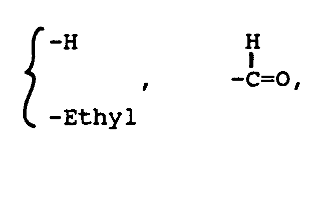 CH₂CH₂C0₂H oder = CHCHO ist;
R₃ Methyl , 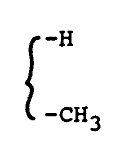 oder 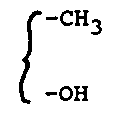 ist;
R₄ Wasserstoff, Vinyl, Ethyl, 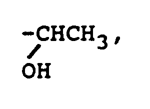 CH₂CH₂C0₂H, = CHCHO oder 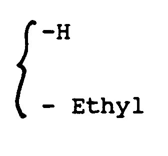 ist;
R₅ Methyl ist;
R₆ Wasserstoff, CH₂ CH₂ COH, CH₂CH₂CO₂R oder CO₂H ist;
R₇ CH₂CH₂CO₂H, CH₂CH₂CO₂R oder 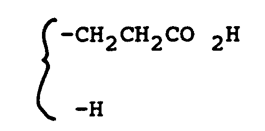 ist;
R₈ Methyl oder 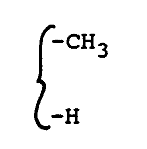 ist;
Rg Wasserstoff, COOH, CH₂, CH₂COOH oder Methyl ist; unter der Voraussetzung, daß, wenn R₁, R₂, R₃, R₄, R₇ und R₈ zwei Substituenten darstellen oder divalent sind und am gleichen Kohlenstoff gebunden ist, der betreffende Pyrrolring, an den sie gebunden sind, ein Dihydropyrrol ist;
R ein niedrigeres Alkyl oder Benzyl ist;
unter der Voraussetzung, daß mindestens einer von Rᵢ - Rg eine freie Carboxylgruppe enthält, und daß zwischen der Aminogruppe der Aminomonocarbonsäure und den Carboxygruppen des Tetrapyrrols Amidbindungen (1 bis 4) gebildet werden; unter der Voraussetzung daß der Tetrapyrrolrest nicht Pheophorbid a ist.

2. Zusammensetzung nach Anspruch 1, in der die Aminosäure eine alpha-Aminosäure ist.
Di--a-Serinyl-trans-mesochlorin IX
Di-L-a-Serinyl-trans-Mesochlorin e₄
Di-L-a-Serinyl-Hematoporphyrin IX
Di-L-a-Serinyl-Protoporphyrin IX
Di-L-a-Serinyl-Deuteroporphyrin
Di-_{E}-Amino-n-Caproyl-Hematoporphyrin IX
Di-_{E}-Amino-n-Caproyl-Chlorin e₆
Di-_{E}-Amino-n-Caproyl-Protoporphyrin IX
Di-_{E}-Amino-n-Caproyl-Deuteroporphyrin
Mono-L-Serinyl-Mesochlorin e₆
Mono-L-Serinyl-Deuterochlorin e₆
Mono-L-Serinyl-2-Formyl-Chlorin e₆
Mono-L-Serinyl-2-Acetyl-Chlorin e₆
Mono-L-Cysteinyl-Chlorin e₆
Mono-L-Asparaginyl-Chlorin e₆
Mono-Serinyl-Chlorin e₆
Mono-(DL)-Glycyl-Chlorin e₆
Alanyl-Chlorin e₆
Mono-L-Valyl-Chlorin e₆
Mono-L-Leucyl-Chlorin e₆
Mono-L-Isoleucyl-Chlorin e₆
Mono-L-Prolyl-Chlorin e₆
Mono-L-Methionyl-Chlorin e₆
Mono-L-Threoninyl-Chlorin e₆
Tyrosyl-Chlorin e₆
Glutaminyl-Chlorin e₆
Lysyl-Chlorin e₆
Arginyl-Chlorin e₆
Histidyl-Chlorin e₆
β-Alanyl-Chlorin e₆
Mono-E-Amino-n-Caproyl-Chlorin e₆
Monoglycyl-Mesoporphyrin IX
Mono-Alanyl-Mesoporphyrin IX
Mono-β-Alanyl-Mesoporphyrin IX
Mono-_{E}-Amino-n-Caproyl-Mesoporphyrin IX
Mono-β-Alanyl-Hematorporphyrin IX
Threoninyl-2-Formylchlorin e₆

3. Zusammensetzung nach Anspruch 1 oder Anspruch 2, in der das Tetrapyrrol ein Porphyrin, Chlorin oder Bacteriochlorin ist.

4. Zusammensetzung nach einem der Ansprüche 1 bis 3, in der die Amid-haltigen Substituenten asymmetrisch am Tetrapyrrol-Molekül angeordnet sind.

5. Zusammensetzung nach Anspruch 1, in der das Amid
Diserinyl-Mesophorphyrin IX
Diglycyl-Mesoporphyrin IX
Di-α-(D,L)-Alanyl-Mesoporphyrin IX
Di-β-Alanyl-Mesoporphyrin IX
Di-_{E}-Amino-n-Caproyl-Mesoporphyrin IX
Diglycyl-trans-Mesochlorin IX
Diglycyl-trans-Mesochlorin e₆
Diglycyl-Mesoschlorin e₄
Diglycyl-Hemaporphyrin IX
Diglycyl-Chlorin e₆
Diglycyl-Protoporphyrin IX
Diglycyl-Deuteroporphyrin
Di-a-(DL)-Alanyl-trans-Mesochlorin IX
Di-a-(DL)-Alanyl-Mesochlorin e₆
Di-a-(DL)-Alanyl-Mesochlorin e₄
Di-a-(DL)-Alanyl-Hematoporphyrin IX
Di-a-(DL)-Alanylchlorin e₆
Di-a-(DL)-Alanyl-Protoporphyrin IX
Di-a-(DL)-Alanyl-Deuteroporphyrin
Di-ß-(DL)-Alanyl-trans-Mesochlorin IX
Di-β-(DL)-Alanyl-Mesochlorin e₆
Di-β-DL)-Alanyl-Mesochlorin e₄
Di-β-(DL)-Alanyl-Hematoporphyrin IX
Di-β-(DL)-Alanyl-Chlorin e₆
Di-β-(DL)-Alanyl-Protoporphyrin IX
Di-L-a-Serinyl-trans-Mesochlorin e₆
Mono-L-Threoninyl-Deuterochlorin e₆ oder
Mono-L-Threoninyl-Mesochlorin e₆
ist.

6. Verwendung eines fluoreszierenden Mono-, Di- oder Polyamids einer Aminomonocarbonsäure und eines Tetrapyrrols, das eine oder zwei Carboxygruppen enthält, gemäß der in einem der Ansprüche 1 bis 5 beanspruchten Struktur, oder eines Salzes davon, und eines pharmazeutisch akzeptablen Trägers dafür, zur Herstellung einer therapeutischen Zusammensetzung für Photodiagnose und/oder für Phototherapie von Tumoren.

## Patentansprüche (Patentansprüche für folgende(n) Vertragsstaat(en) : AT)

1. Verwendung eines fluoreszierenden Mono-, Di- oder Polyamids einer Aminomonocarbonsäure und einer Tetrapyrrol-Verbindung der Formel 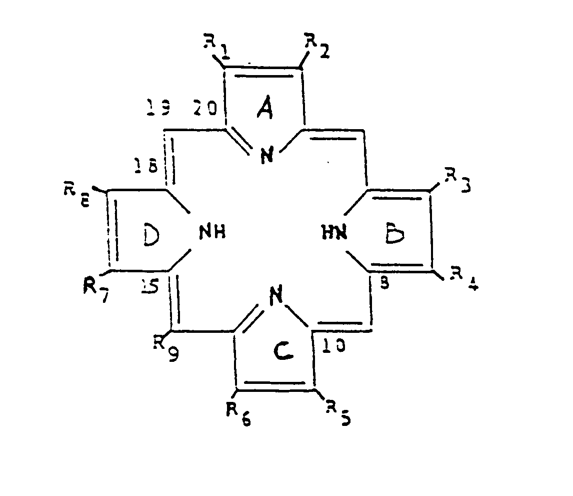 oder eines Salzes davon,
oder der entsprechenden Di- oder Tetrapyrrole und eines pharmazeutisch akzeptablen Trägers dafür, wobei
R₁ Methyl; 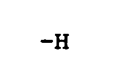 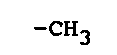 oder 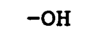 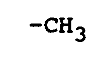 ist;
R₂ Wasserstoff, Vinyl, Ethyl, 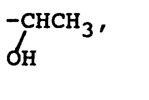 Acetyl, 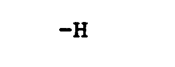 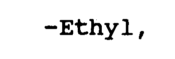 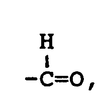 -CH₂CH₂CO₂H oder = CHCHO ist;
R₃ Methyl, 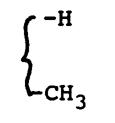 oder 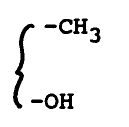 ist;
R₄ Wasserstoff, Vinyl, Ethyl, 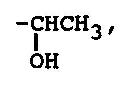 -CH₂CH₂CO₂H, = CHCHO oder 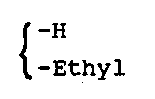 ist;
R₅ Methyl ist;
R₆ Wasserstoff, -CH₂CH₂CO₂ H, CH₂CH₂CO₂ R oder C0₂ H ist;
R₇ CH₂CH₂CO₂H, CH₂CH₂C0₂R oder 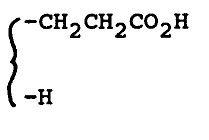 ist;
R₈ Methyl oder 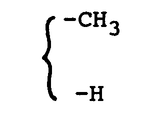 ist;
R₉ Wasserstoff, COOH, CH_{2COO}H oder Methyl ist;
unter der Voraussetzung, daß, wenn Rᵢ, R₂, R₃, R₄, R₇ und R₈ zwei Substituenten darstellen oder divalent und am gleichen Kohlenstoff gebunden sind, der betreffende Pyrrolring, an den sie gebunden sind, ein Dihydropyrrol ist;
R ist ein niedrigeres Alkyl oder Benzyl,
unter der Voraussetzung, daß mindestens einer von R₁ bis R₉ eine freie Carboxylgruppe enthält, und daß zwischen der Aminogruppe der Aminomonocarbonsäure und einer der Carboxygruppen des Tetrapyrrols Amidverbindungen (1 bis 4) gebildet werden; unter der Voraussetzung, daß der Tetrapyrrolring nicht Pheophorbid a ist;
zur Herstellung einer therapeutischen Zusammensetzung für Fotodiagnose und/oder die Fototherapie von Tumoren.

2. Verwendung gemäß Anspruch 1,
dadurch gekennzeichnet,
daß die Aminosäure eine alpha-Aminosäure ist.

## Revendications (Revendications pour l'(les) Etat(s) contractant(s) suivant(s) : BE, CH, DE, FR, GB, IT, LI, LU, NL, SE)

1. Composition thérapeutique comprenant un mono-, di- ou polyamide fluorescent d'un acide aminomonocarboxylique et d'un composé de tétrapyrrole représenté par la formule : 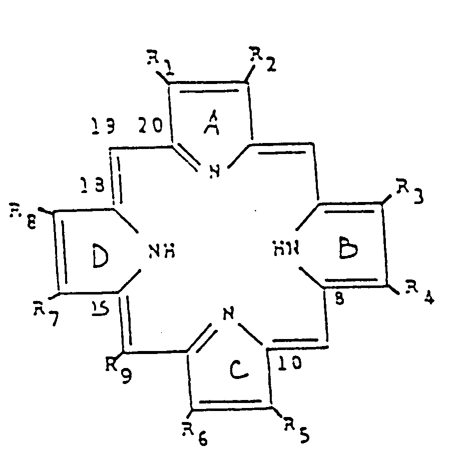 ou un sel de celui-ci,
ou les di- ou tétrahydrotétrapyrroles correspondants ; et
un support pharmaceutiquement acceptable pour celui-ci ; où :
- R₁ représente méthyle, 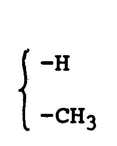 ou 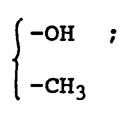
- R₂ représente H, vinyle, éthyle, 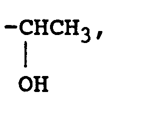 acétyle, 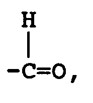 CH₂CH₂CO₂H, ou =CHCHO ; 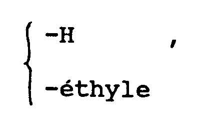
- R₃ représente méthyle, 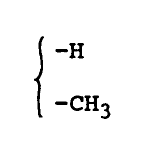 ou 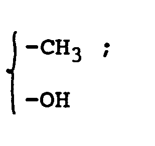
- R₄ représente H, vinyle, éthyle, 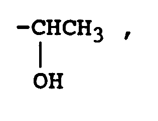 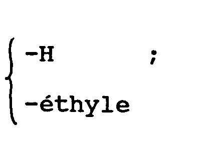
- R₅ représente méthyle ;
- R₆ représente H, CH₂ CH₂ CO₂ H, CH₂ CH₂ CO₂ R ou CO₂ H ;
- R₇ représente CH₂CH₂C_{O2} H,CH₂ CH₂ CO₂ R, ou 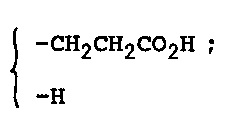
- R₈ représente méthyle ou 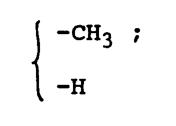
- R₉ représente H, COOH, CH₂COOH ou méthyle ;
à la condition que, lorsque R₁, R₂, R₃, R₄, R₇ et R₈ représentent deux substituants ou sont bivalents et attachés au même carbone, le noyau pyrrole respectif auquel ils sont attachés est un dihydropyrrole ;
- R représente alkyle inférieur ou benzyle ;
avec la condition qu'au moins l'un parmi R₁ -R₉ comprenne un groupe carboxyle libre, et que des liaisons amide (1 à 4) soient formées entre le groupe amino de l'acide aminomonocarboxylique et l'un des groupes carboxyle du tétrapyrrole : avec la condition que le reste tétrapyrrole ne représente pas un phéophorbide a.

2. Composition selon la revendication 1, dans laquelle l'acide aminé est un alpha amino acide.

3. Composition selon l'une des revendications 1 et 2, dans laquelle, le tétrapyrrole est une porphyrine, une chlorine ou une bactériochlorine.

4. Composition selon l'une quelconque des revendications 1 à 3, dans laquelle les substituants contenant un amide sont disposés de manière asymétrique sur la molécule de tétrapyrrole.

5. Composition selon la revendication 1, dans laquelle l'amide est :
- la disérinyl mésoporphyrine IX ;
- la diglycyl mésoporphyrine IX ;
- la di-a-(DL)-alanyl mésoporphyrine IX ;
- la di-β-alanyl mésoporphyrine IX ;
- la di-_{E}-amino-n-caproyl mésoporphyrine IX ;
- la diglycyl trans-mésochlorine IX ;
- la diglycyl trans-mésochlorine e6 ;
- la diglycyl mésochlorine e4 ;
- la diglycyl hématoporphyrine IX ;
- la diglycyl chlorine e₆ ;
- la diglycyl protoporphyrine IX ;
- la diglycyl deutéroporphyrine ;
- la di-a-(DL)-alanyl trans-mésochlorine IX ;
- la di-a-(DL)-alanyl mésochlorine es ;
- la di-a-(DL)-alanyl mésochlorine e4 ;
- la di-a-(DL)-alanyl hématoporphyrine IX ;
- la di-a-(DL)-alanyl chlorine e₆ ;
- la di-a-(DL)-alanyl protoporphyrine IX ;
- la di-a-(DL)-alanyl deutéroporphyrine ;
- la di-β-(DL)-alanyl trans-mésochlorine IX ;
- la di-β-(DL)-alanyl mésochlorine es ;
- la di-β-(DL)-alanyl mésochlorine e4 ;
- la di-β-(DL)-alanyl hématoporphyrine IX ;
- la di-β-(DL)-alanyl chlorine e₆ ;
- la di-β-(DL)-alanyl protoporphyrine IX ;
- la di-β-(DL-alanyl deutéroporphyrine ;
- la di-L-a-sérinyl chlorine e₆ ;
- la di-L-a-sérinyl trans-mésochlorine e₆
- la di-L-a-sérinyl trans-mésochlorine IX ;
- la di-L-a-sérinyl trans-mésochlorine e4 ;
- la di-L-a-sérinyl hématoporphyrine IX ;̅
- la di-L-a-sérinyl protoporphyrine IX ;
- la di-L-a-sérinyl deutéroporphyrine ;
- la di-_{E}-amino-n-caproyl-hématoporphyrine IX ;
- la di-_{E}-amino-n-caproyl-chlorine e₆ ;
- la di-_{E}-amino-n-caproyl-protoporphyrine IX ;
- la di-ε-amino-n-caproyl-deutéroporphyrine ;
- la mono-L-sérinyl mésochlorine e₆ ;
- la mono-L-sérinyl deutérochlorine e₆
- la mono-L-sérinyl-2-formyl chlorine e₆ ;
- la mono-L-sérinyl-2-acétyl chlorine e₆
- la mono-L-cystéinyl chlorine e₆ ;
- la mono-L-asparaginyl chlorine e₆
- la mono sérinyl chlorine e₆ ;
- la mono-(DL) glycyl chlorine e₆
- l'alanyl chlorine e₆ ;
- la mono-L-valyl chlorine e₆ ;
- la mono-L-leucyl chlorine e₆ ;
- la mono-L-isoleucyl chlorine e₆ ;
- la mono-L-prolyl chlorine es ;
- la mono-L-méthionyl chlorine e₆ ;
- la mono-L-thréoninyl chlorine e₆ ;
- la tyrosyl chlorine e₆ ;
- la glutaminyl chlorine e₆ ;
- la lysyl chlorine e₆ ;
- l'arginyl chlorine e₆ ;
- l'histidyl chlorine e₆ ;
- la β-alanyl chlorine e₆ ;
- la mono-_{E}-amino-n-caproyl chlorine e₆ ;
- la monoglycyl mésoporphyrine IX ;
- la mono alanyl mésoporphyrine IX ;
- la mono-β-alanyl mésoporphyrine IX ;
- la mono-E-amino-n-caproyl mésoporphyrine IX ;
- la mono-β-alanyl-hématoporphyrine IX ;
- la thréoninyl-2-formylchlorine e₆ ;
- la mono-L-thréoninyl deuterochlorine e6 ; ou
- la mono-L-thréoninyl mésochlorine eₛ.

6. Utilisation d'un mono-, di- ou polyamide fluorescent d'un acide aminomonocarboxylique et d'un tétrapyrrole contenant un ou deux groupes carboxy de la structure telle que définie à l'une quelconque des revendications 1 à 5, ou d'un sel de celui-ci, et d'un support pharmaceutiquement acceptable pour celui-ci, pour la préparation d'une composition thérapeutique pour le photodiagnostic et/ou la photothérapie de tumeurs.

## Revendications (Revendications pour l'(les) Etat(s) contractant(s) suivant(s) : suivant : AT)

1. Utilisation d'un mono-, di- ou polyamide fluorescent d'un acide aminomonocarboxylique et d'un composé de tétrapyrrole représenté par la formule : 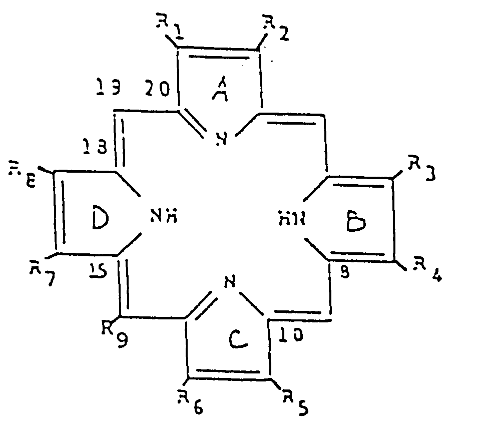 ou d'un sel de celui-ci,
ou des di- ou tétrahydrotétrapyrroles correspondants ; et d'un support pharmaceutiquement acceptable pour celui-ci ; où :
- R₁ représente méthyle, 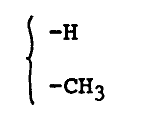 ou 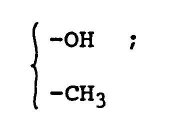
R₂ représente H, vinyle, éthyle, 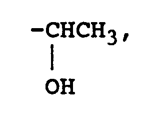 acétyle, 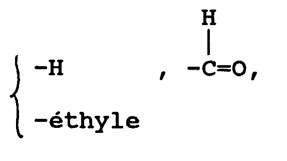 CH₂CH₂CO₂H, ou =CHCHO ;
- R₃ représente méthyle, 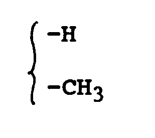 ou 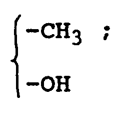
- R₄ représente H, vinyle, éthyle, 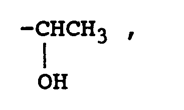 CH₂CH₂CO₂H, =CHCHO, ou 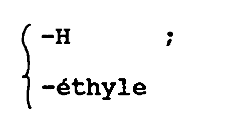
- R₅ représente méthyle ;
- R₆ représente H, CH₂ CH₂ CO₂ H, CH₂CH₂CO₂ R ou C0₂ H :
- R₇ représente CH₂ CH₂ CO₂ H,CH₂ CH₂ CO₂ R, ou 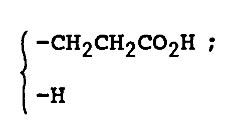
- R₈ représente méthyle ou 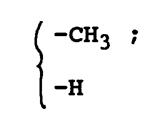
- Rg représente H, COOH, CH₂COOH ou méthyle ;
à la condition que, lorsque R₁, R₂, R₃, R₄, R₇ et R₈ représentent deux substituants ou sont bivalents et attachés au même carbone, le noyau pyrrole respectif auquel ils sont attachés est un dihydropyrrole ;
- R représente alkyle inférieur ou benzyle ;
avec la condition qu'au moins l'un parmi R₁ -R9 comprenne un groupe carboxyle libre, et que des liaisons amide (1 à 4) soient formées entre le groupe amino de l'acide aminomonocarboxylique et l'un des groupes carboxyle du tétrapyrrole : avec la condition que le reste tétrapyrrole ne représente pas un phéophorbide a.
pour la préparation d'une composition thérapeutique pour le photodiagnostic et/ou la photothérapie de tumeurs.

2. Utilisation selon la revendication 1, caractérisée par le fait que l'acide aminé est un alpha amino acide.
